# EUROPEAN PATENT SPECIFICATION

(11) **EP 3 713 590 B1**
(45) Date of publication and mention of the grant of the patent: **07.05.2025**
(21) Application number: 18803442.5
(22) Date of filing: 21.11.2018
(51) Int. Cl.: A61K 38/17, A61P 37/06

(54) **HUMAN BETA 2 DEFENSIN FOR USE IN THE PREVENTION AND TREATMENT OF GRAFT-VERSUS-HOST-DISEASE**
HUMANES BETA 2 DEFENSIN ZUR VERWENDUNG IN DER VORBEUGUNG UND BEHANDLUNG DER TRANSPLANTAT-WIRT-REAKTION
BETA 2 DÉFENSINE HUMAINE POUR L'UTILISATION DANS LA PRÉVENTION ET TRAITEMENT DE LA RÉACTION DU GREFFON CONTRE L'HÔTE

(30) Priority: 24.11.2017 EP 17203527; 31.08.2018 EP 18191954
(43) Date of publication of application: 30.09.2020
(73) Proprietor: Defensin Therapeutics ApS, 2200 København N (DK)
(72) Inventor: NORDKILD, Peter, 1316 København K (DK)
(74) Representative: Høiberg P/S
(86) International application number: PCT/EP2018/082022
(87) International publication number: WO 2019/101773

(56) References cited:
- WO-A1-2017/129195
- WO-A2-2019/092201
- WO-A2-2019/092201
- DANIELA WEBER ET AL: "The association between acute graft-versus-host disease and antimicrobial peptide expression in the gastrointestinal tract after allogeneic stem cell transplantation", PLOS ONE, vol. 12, no. 9, 21 September 2017 (2017-09-21), pages e0185265, XP055552032, DOI: 10.1371/journal.pone.0185265
- NITA H SALZMAN ET AL: "Enteric defensins are essential regulators of intestinal microbial ecology", NATURE IMMULOGY, vol. 11, no. 1, 22 October 2009 (2009-10-22), New York, pages 76 - 82, XP055360095, ISSN: 1529-2908, DOI: 10.1038/ni.1825
- Y. ERIGUCHI ET AL: "Graft-versus-host disease disrupts intestinal microbial ecology by inhibiting Paneth cell production of -defensins", BLOOD, vol. 120, no. 1, 5 July 2012 (2012-07-05), US, pages 223 - 231, XP055551825, ISSN: 0006-4971, DOI: 10.1182/blood-2011-12-401166
- A. BISWAS ET AL: "Induction and rescue of Nod2-dependent Th1-driven granulomatous inflammation of the ileum", PROCEEDINGS OF THE NATIONAL ACADEMY OF SCIENCES, vol. 107, no. 33, 2 August 2010 (2010-08-02), pages 14739 - 14744, XP055552099, ISSN: 0027-8424, DOI: 10.1073/pnas.1003363107
- DANIELA WEBER ET AL: "The association between acute graft-versus-host disease and antimicrobial peptide expression in the gastrointestinal tract after allogeneic stem cell transplantation", PLOS ONE, vol. 12, no. 9, 21 September 2017 (2017-09-21), pages e0185265, XP055552032, DOI: 10.1371/journal.pone.0185265
- NITA H SALZMAN ET AL: "Enteric defensins are essential regulators of intestinal microbial ecology", NATURE IMMULOGY, vol. 11, no. 1, 22 October 2009 (2009-10-22), New York, pages 76 - 82, XP055360095, ISSN: 1529-2908, DOI: 10.1038/ni.1825
- Y. ERIGUCHI ET AL: "Graft-versus-host disease disrupts intestinal microbial ecology by inhibiting Paneth cell production of -defensins", BLOOD, vol. 120, no. 1, 5 July 2012 (2012-07-05), US, pages 223 - 231, XP055551825, ISSN: 0006-4971, DOI: 10.1182/blood-2011-12-401166
- A. BISWAS ET AL: "Induction and rescue of Nod2-dependent Th1-driven granulomatous inflammation of the ileum", PROCEEDINGS OF THE NATIONAL ACADEMY OF SCIENCES, vol. 107, no. 33, 2 August 2010 (2010-08-02), pages 14739 - 14744, XP055552099, ISSN: 0027-8424, DOI: 10.1073/pnas.1003363107
- EDWARD K. GEISSLER ET AL: "KFO 243 Final Report ELITE: Early Immunological Determinants of Late Transplant Outcome", 21 February 2018 (2018-02-21), pages 1 - 124, XP055552021, Retrieved from the Internet <URL:http://www-kfo.uni-regensburg.de/kfo243/pdf/KFO243_ELITE_FINAL%20REPORT_21FEB2018_FINAL.pdf> [retrieved on 20190205]
- DOROTHY M SUPP: "Gene therapy to fight infection in skin transplants", PHARMACOGENOMICS, vol. 8, no. 5, 1 May 2007 (2007-05-01), UK, pages 483 - 486, XP055551698, ISSN: 1462-2416, DOI: 10.2217/14622416.8.5.483
- HAKMO LEE ET AL: "Granulocyte-Derived Cationic Peptide Enhances Homing and Engraftment of Bone Marrow Stem Cells after Transplantation", LABORATORY ANIMAL RESEARCH, vol. 27, no. 2, 1 January 2011 (2011-01-01), pages 133, XP055551836, ISSN: 1738-6055, DOI: 10.5625/lar.2011.27.2.133
- EDWARD K. GEISSLER ET AL: "KFO 243 Final Report ELITE: Early Immunological Determinants of Late Transplant Outcome", 21 February 2018 (2018-02-21), pages 1 - 124, XP055552052, Retrieved from the Internet <URL:http://www-kfo.uni-regensburg.de/kfo243/pdf/KFO243_ELITE_FINAL%20REPORT_21FEB2018_FINAL.pdf> [retrieved on 20190205]
- D. MAILÄNDER-SÁNCHEZ ET AL: "DOP083 Recombinant subcutaneous human beta-Defensin 2 (hBD2) ameliorates experimental colitis in different in vivo models", JOURNAL OF CROHN'S AND COLITIS, vol. 11, no. suppl_1, 1 February 2017 (2017-02-01), NL, pages S75 - S76, XP055552046, ISSN: 1873-9946, DOI: 10.1093/ecco-jcc/jjx002.120
- L KOENINGER ET AL: "P851 Oral delivery of Human [beta]-defensin 2 is reversibly increasing microbiome diversity and is effective in the treatment of experimental colitis", JOURNAL OF CROHN'S AND COLITIS, vol. 12, no. supplement_1, 16 January 2018 (2018-01-16), NL, pages S547 - S547, XP055552067, ISSN: 1873-9946, DOI: 10.1093/ecco-jcc/jjx180.978
- EIKO HAYASE ET AL: "R-Spondin1 expands Paneth cells and prevents dysbiosis induced by graft-versus-host disease", THE JOURNAL OF EXPERIMENTAL MEDICINE, vol. 214, no. 12, 24 October 2017 (2017-10-24), US, XP055552211, ISSN: 0022-1007, DOI: 10.1084/jem.20170418
- M INOUE ET AL: "Colostrum and severe gut GVHD", BONE MARROW TRANSPLANTATION VOLUME, vol. 22, 4 August 1998 (1998-08-04), pages 402 - 403, XP055552260
- JOANNA BARICELLI ET AL: "[beta]-defensin-2 in breast milk displays a broad antimicrobial activity against pathogenic bacteria", JORNAL DE PEDIATRIA, vol. 91, no. 1, 1 January 2015 (2015-01-01), BR, pages 36 - 43, XP055551294, ISSN: 0021-7557, DOI: 10.1016/j.jped.2014.05.006
- ZHENWEI SHEN ET AL: "[beta]-Defensin 2 Ameliorates Lung Injury Caused by Pseudomonas Infection and Regulates Proinflammatory and Anti-Inflammatory Cytokines in Rat", INT. J. MOL. SCI., vol. 15, no. 8, 30 July 2014 (2014-07-30), pages 13372 - 13387, XP055461387, ISSN: 1661-6596, DOI: 10.3390/ijms150813372
- ARMOGIDA SHEILA A ET AL: "Identification and quantification of innate immune system mediators in human breast milk", ALLERGY AND ASTHMA PROCEEDI, PROVIDENCE, RI, US, vol. 25, no. 5, 1 September 2004 (2004-09-01), pages 297 - 304, XP009150956, ISSN: 1088-5412

## Description

### Field of the invention

The present invention relates to a human β-defensin selected from hBD-2 (SEQ ID NO.: 2), truncated hBD-2 (SEQ ID NO.: 7) or a functionally equivalent modified defensin comprising 1-4 amino acid modifications compared to hBD-2 of SEQ ID NO.: 2 or truncated hBD-2 of SEQ ID NO.: 7; wherein said modification(s) is a conservative amino acid substitution(s) or insertion(s) that do not significantly affect the folding of the defensin; or a single deletion(s), for use in the prevention or treatment of acute graft-versus-host-disease (GVHD) in a patient that has received or is about to receive an allogeneic hematopoietic stem cell transplantation. The invention can lead to treatment of symptoms of GVHD such as diarrhoea and sepsis based on normalization of gut and lung microbiota and rebalancing of the immune system with reduced cytokine production eliminating the risk of a cytokine storm.

### Background

Graft-versus-host-disease continues to be a leading cause of morbidity and mortality after allogeneic hematopoietic stem cell transplantation. Gastrointestinal GVHD is the major cause of mortality (Teshima, 2016). The initial phase of acute GVHD is triggered by tissue damage and associated loss of mucosal barrier function, primarily in the gastrointestinal tract, which is caused by the conditioning regimens needed to bring malignant disease to a minimal residual level suitable for subsequent immune control and to ablate existing immune function, allowing engraftment of the naïve donor inoculum. Stem cell transplantation typically uses total body irradiation, immunosuppression and chemotherapy to achieve these dual aims; however, they also result in damage to the GI tract mucosa and other cells contributing to the "cytokine storm", which is characterized by the release of proinflammatory cytokines; classically Th1 cytokines: IFNγ, IL-2 and TNF-α and Th2 cytokines: IL-4, IL-5, IL-10 and IL-13 (Henden, 2015). Current mainstay GVHD therapies focus on broad immunosuppression, which can affect engraftment and, in some contexts, reduce the desired graft-versus-tumor activity (Nalle, 2015). A reduction in the bacterial burden by use of antimicrobial decontamination in the posttransplant period can reduce GVHD severity. The lung and the skin are the primary target organs in chronic GVHD, manifesting as fibrosis - bronchiolitis obliterans and scleroderma (Henden, 2015). Acute GVHD remains a major cause of treatment failure resulting in mortality in 20% of recipients and 60-80% of survivors experience some degree of chronic GVHD (Markey, 2014). < insert page 2a>

### Summary

The invention is defined by the claims. Any subject matter falling outside the scope of the claims is provided for information purposes only.

The inventor has surprisingly demonstrated in a mouse model of acute graft-versus-host disease arising from allogenic hematopoietic stem cell transplantation that a prophylactic dose of oral hBD2 or HD5 is capable of decreasing mortality dramatically and to a larger extent than cyclosporine, the most commonly used treatment of GVHD. It was also demonstrated in this model that hBD2 was able to decrease the production of IL-1β in myeloid cells (CD11c+ dendritic cells and CD11b+ Ly6FG- cells). IL-1β being a major driver of intestinal GvHD.

The inventor has also surprisingly demonstrated in both preventive and therapeutic mouse models of severe intestinal inflammation (DSS, TNBS, T-cell transfer), hBD2 mitigates diarrhoea and weight loss through improved intestinal health and decreases the disease activity index on par with standard immunosuppressants such as prednisolone/dexamethasone and cyclosporin as well as anti TNF-α, all routinely used in the prevention and/or treatment of GVHD.

It has surprisingly been demonstrated that hBD2 can rebalance the immune system through normalization of cytokine production of IFN-γ, TNF-α, IL-4, IL-5, IL-6, IL-9, IL-10 and IL-13. While some drugs e.g. anti TNF-α can normalize one or a few cytokines, no other drugs have been identified that can normalize all of these cytokines and prevent a cytokine storm. Inflammatory cytokine release or cytokine storm that has been implicated as the primary mediator of the occurrence and severity of acute GVHD (Ball & Egeler, 2008).

Further, it has been demonstrated in both a preventive and a therapeutic mouse model that both HD5 and hBD2 have a strong influence on the composition of the microbiota. For example defensins can increase the variety of microbes i.e. improve/normalize the microbiota but also that defensins can increase the abundance of short chain fatty acid producing bacteria. Short chain fatty acids, that play a key role in colonic T reg cell homeostasis, that is perturbed in acute GVHD. The effects of defensins are different from one defensin to another and the combination of an α- and a β-defensin is yet again different from the individual effects.

Hyase et al (J Exp Med 2017 Vol 214, pp 3507-3518) relates to the Wnt agonist R-spodin 1 (R-Spo 1) and the mouse α-defensin cryptidin-4 (Crp4) and their respective use in restoration of intestinal microbial ecology and the prevention of GVHD-mediated dysbiosis. The document shows that both R-Spo 1 and orally administered Crp4 have positive effects on the microbiome. However, the document concludes that treatment with the α-defensin Crp4 did not show a significant effect on survival of the mice.

It has further surprisingly been demonstrated in a mouse model of acute asthma that a dosage of human beta-defensin 2 (hBD2) whether administered orally or intranasally is capable of preventing the rise of a cytokine storm and the development of asthma as well as improve lung function dramatically. Contrary to conventional asthma prophylaxis, where general immune suppression is the target, prophylaxis with hBD2 rebalances the immune system.

It has further surprisingly been demonstrated in a mouse model of acute asthma that a dosage of human beta-defensin 2 (hBD2) whether administered orally or intranasally is also capable of treating asthma normalizing airway hyper-responsiveness and compliance.

In conclusion, the inventor has demonstrated that HD5 and hBD2 have all the properties required in order to be a safe and efficacious treatment of complications arising from allogenic hematopoietic stem cell transplantation. The peptides are safe as they are endogenous, physiological, human peptides. Defensins can be used in amounts not exceeding the amount present in colostrum received by a full term newborn infant. Defensins can be administered through oral, subcutaneous or intrapulmonary administration. Defensins can rebalance the immune system without suppressing the immune system. Defensins can prevent a cytokine storm, the major culprit of acute GVHD. Defensins can normalise the microbiota and metabolome and promote production of short chain fatty acids, that preserves colonic T reg cell homeostasis. Defensins can treat severe intestinal, liver and lung inflammation.

Therefore in one aspect, the invention relates to a human β-defensin selected from hBD-2 (SEQ ID NO.: 2), truncated hBD-2 (SEQ ID NO.: 7) or a functionally equivalent modified defensin comprising 1-4 amino acid modifications compared to hBD-2 of SEQ ID NO.: 2 or truncated hBD-2 of SEQ ID NO.: 7; wherein said modification(s) is a conservative amino acid substitution(s) or insertion(s) that do not significantly affect the folding of the defensin; or a single deletion(s), for use in the prevention or treatment of acute graft-versus-host-disease (GVHD) in a patient that has received or is about to receive an allogeneic hematopoietic stem cell transplantation

The patient may have severe intestinal and liver inflammation (e.g. characterised by abdominal pain, nausea, vomiting, and jaundice) and loss of mucosal defence, cytokine storm, weight loss, or sepsis, skin rash, itching, and redness.

The effect of the treatment may comprise rebalancing of the immune system and preventing or treating the cytokine storm through normalization of the tissue cytokine production.

The effect of the treatment may comprise treatment of gut permeability and sepsis through normalization of the mucosal defence or myeloperoxidase activity in the intestines.

The effect of the treatment may comprise treatment of respiratory complications, lung inflammation and sepsis.

The effect of the treatment may comprise reducing histological lung inflammation, peribronchial, and perivascular inflammation as well as and inflammatory cell migration into bronchoalveolar lavage fluid.

The effect of the treatment may comprise treatment and/or prevention of inflammation and fibrosis of the lungs and skin, such as bronchiolitis obliterans and scleroderma.

The effect of the treatment may comprise increasing gene richness, increasing the number of phylae, increasing the bacterial presence, increasing the bacterial abundance and/or increasing the short chain fatty acid production, butyrate production and/or decreasing the acetate production from gut or lung microbiota of said patient.

The effect of the treatment may comprise maturing, maintaining and/or stabilizing a normal microbiota in the gut or lung or skin of said patient.

The effect of the treatment may comprise increasing the abundance of Allobaculum, Alloprevotella, Akkermansia, Barnesiella, Bifidobacteriaceae, Faecalibacterium, Lachnospira, Rothia and Veillonella in the gut or lung of said patient.

The effect of the treatment may comprise increasing food uptake and weight gain in said patient.

The human β-defensin for use according to claim 1 may be selected from a group consisting of hBD2 or truncated hBD-2 (SEQ ID NO.: 7).

The human β-defensins for use according to claim 1 may be administered alone or in a composition, wherein said composition comprises more than one defensin, such as two defensins, such as three defensins, such as four defensins, such as five defensins. Preferably the two defensins are hBD2 and HD5.

The human β-defensin for use according to claim 1 may further comprise at least one additional moiety selected from a group consisting of a cell penetrating peptide (CPP), an Albumin Binding Moiety (ABM), a detectable moiety (Z), and a half-life extending peptide.

The human β-defensin for use according to claim 1 may be administered alone or in combination with prebiotics, probiotics, tryptophane, short chain fatty acids, glucocorticoids, cyclosporine, anti TNF-α, integrins, antibiotics, immunosuppressants, faecal transplantation, irradiation or a combination of these.

### Description of Drawings

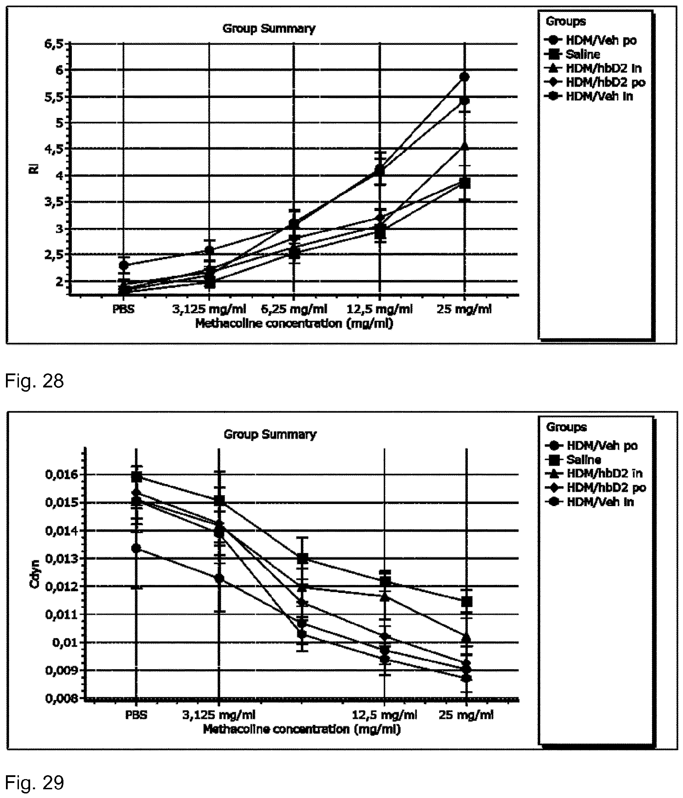

Figure 1 (for illustration). Schematic outline of the experimental setup for investigating the effect of defensins in a Dextran Sodium Sulphate prophylactic model of colitis.
Figure 2 (for illustration). Schematic outline of the experimental setup for investigating the effect of defensins in a Dextran Sodium Sulphate therapeutic model of colitis.
Figure 3 (for illustration). Schematic outline of the experimental setup for investigating the effects of mammalian defensins (HD5, hBD2 and HD5+hBD2) on the composition of the microbiota in a high fat diet murine model.
Figure 4 (for illustration). Schematic outline of the experimental set up for investigating the effects of mammalian defensins in a murine steroid-sensitive model for prevention of asthma, where the mice are immunized by house dust mite (HDM) + Freund's adjuvant and challenged with HDM.
Figure 5 (for illustration). Schematic outline of the experimental set up for investigating the effects of mammalian defensins in a murine steroid-sensitive model for treatment of asthma, where the mice are immunized by house dust mite (HDM) + Freund's adjuvant and challenged with HDM.
Figure 6a. Clustal W (2.1) multiple sequence alignment of human beta defensin 1-4: In the Clustal W alignments:
   *
      indicates positions which have a single, fully conserved residue.
      indicates that one of the following 'strong' groups is fully conserved: -S,T,A; N,E,Q,K; N,H,Q,K; N,D,E,Q; Q,H,R,K; M,I,L,V; M,I,L,F; H,Y; F,Y,W.
      indicates that one of the following 'weaker' groups is fully conserved: -C,S,A; A,T,V; S,A,G; S,T,N,K; S,T,P,A; S,G,N,D; S,N,D,E,Q,K; N,D,E,Q,H,K; N,E,Q,H,R,K; V,L,I,M; H,F,Y.

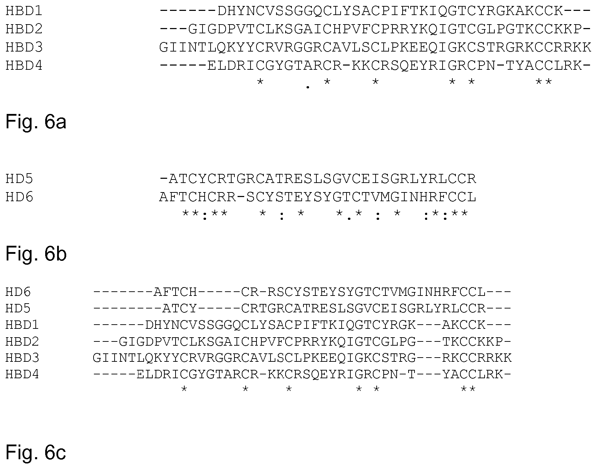

Figure 6b (for illustration). Clustal alignment of HD5 and HD6.
Figure 6c. Clustal alignment of HD5, HD6, and hBD1, hBD2, hBD3, and hBD4.

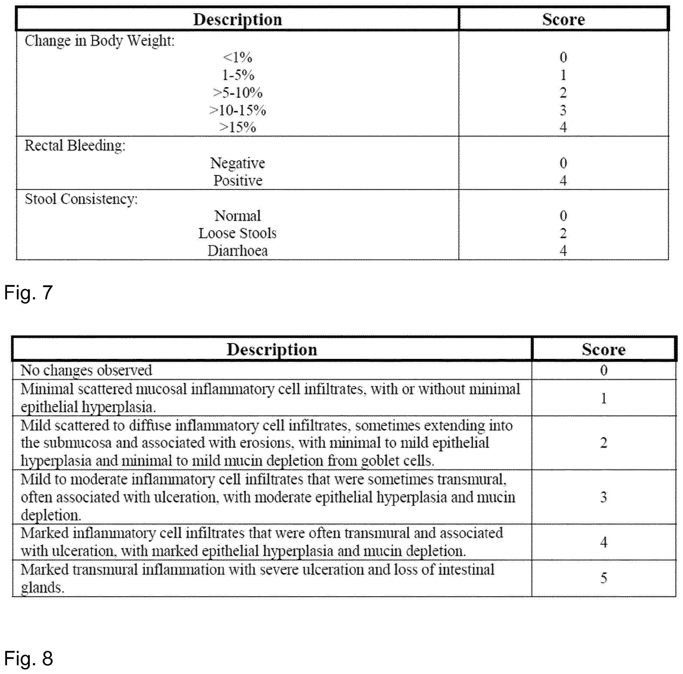

Figure 7 (for illustration). Description of the Disease Activity Index Scoring System for colitis studies
Figure 8 (for illustration). Description of the Histological Scoring System for colitis studies.

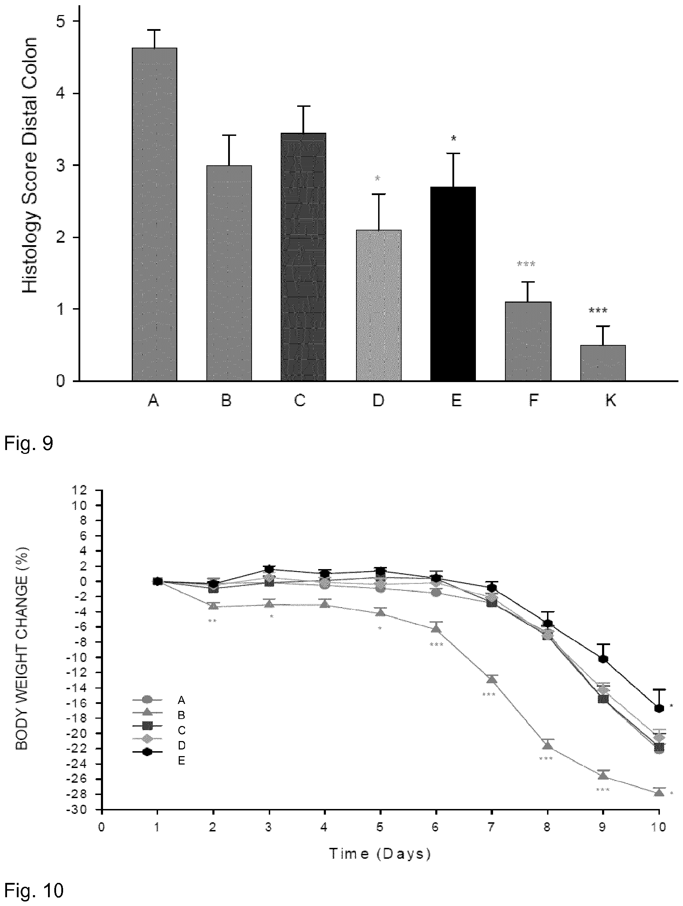

Figure 9 (for illustration). Histological score of distal colon in murine 10-day prophylactic dextran sodium sulphate induced colitis study showing statistically significant superiority of HBD2 (NZ39000) over mouse anti TNF-α and subcutaneous hBD2 administration showing superiority over i.v. administration of HBD2. Legend:
   A: Control vehicle i.v. once per day
   B: Anti-TNF alpha (300 µg/mouse i.p. 3 times)
   C: hBD2 (0.1 mg/kg i.v. once per day)
   D: hBD2 (0.1 mg/kg s.c. once per day)
   E: hBD2 (0.1 mg/kg i.v.. + s.c. twice per day)
   F: hBD2 (0.1 mg/kg s.c. + s.c. twice per day)
   K: naive animals.
Figure 10 (for illustration). Animal body weight change in a murine 10-day prophylactic dextran sodium sulphate induced colitis study showing a weight preserving effect for all doses of orally administered HBD2hBD2 versus 1 mg/kg oral prednisolone. Treatment groups: A: control vehicle PBS twice per day p.o. B: prednisolone 1mg/kg twice per day p.o. C: hBD2 twice per day at 0.05 mg/kg p.o. D: hBD2 twice per day at 0.5 mg/kg p.o.; E: hBD2 twice per day at 5 mg/kg p.o. Significant differences from control (vehicle) group values are shown as **P<0,01; ***P<0,001 (Kruskal-Wallis Test + post -test of Dunn for non-parametric data).

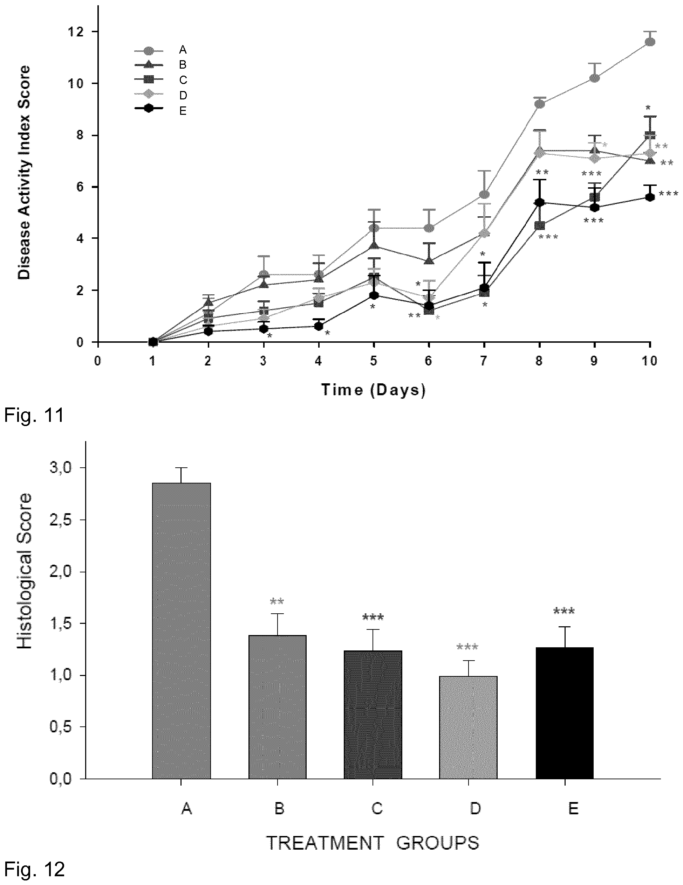

Figure 11 (for illustration). Disease activity index score in a murine 10-day prophylactic dextran sodium sulphate induced colitis study showing statistically significant superiority of orally administered hBD2 over prednisolone 1 mg/kg. Treatment groups and significance shown as for Figure 10.
Figure 12 (for illustration). Histological score in a murine 10-day prophylactic dextran sodium sulphate induced colitis study showing statistical significant effect of all doses of oral HBD2 on par with prednisolone 1 mg/kg. Treatment groups and significance shown as for Figure 10.

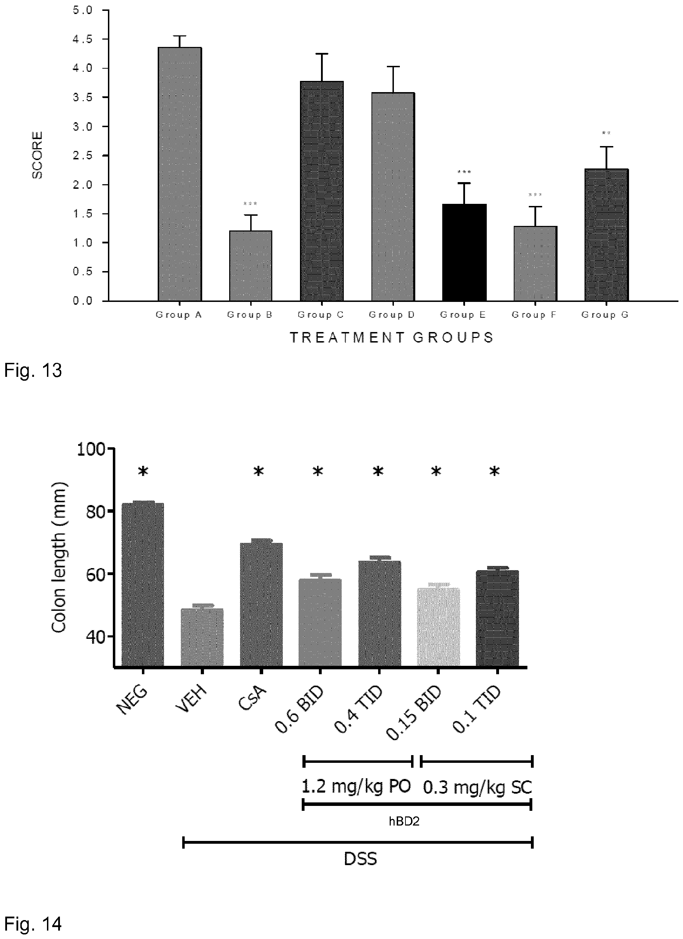

Figure 13 (for illustration). Histological score in an 8-day prophylactic Trinitrobenzene sulfonic acid induced colitis study showing statistical significant effect of 0.03 and 0.1 mg/kg hBD2 (Group E and F) on par with prednisolone 10 mg/kg.
   A: Vehicle control s.c. 1 x daily
   B: prednisolone 10 mg/kg s.c. 1 x daily
   C: hBD2, s.c. 1 x daily = 1 mg/kg
   D: hBD2, s.c. 1 x daily = 0.3 mg/kg
   E: hBD2, s.c. 1 x daily = 0.1 mg/kg
   F: hBD2, s.c. 1 x daily = 0.03 mg/kg
   G: hBD2, s.c. 1 x daily = 0.1 mg/kg
Figure 14 (for illustration). Colon length in a murine 7-day prophylactic dextran sodium sulphate induced colitis study showing statistical significant effect of hBD2 on par with cyclosporine. *p<0.05 vs DSS, Mann Whitney test.

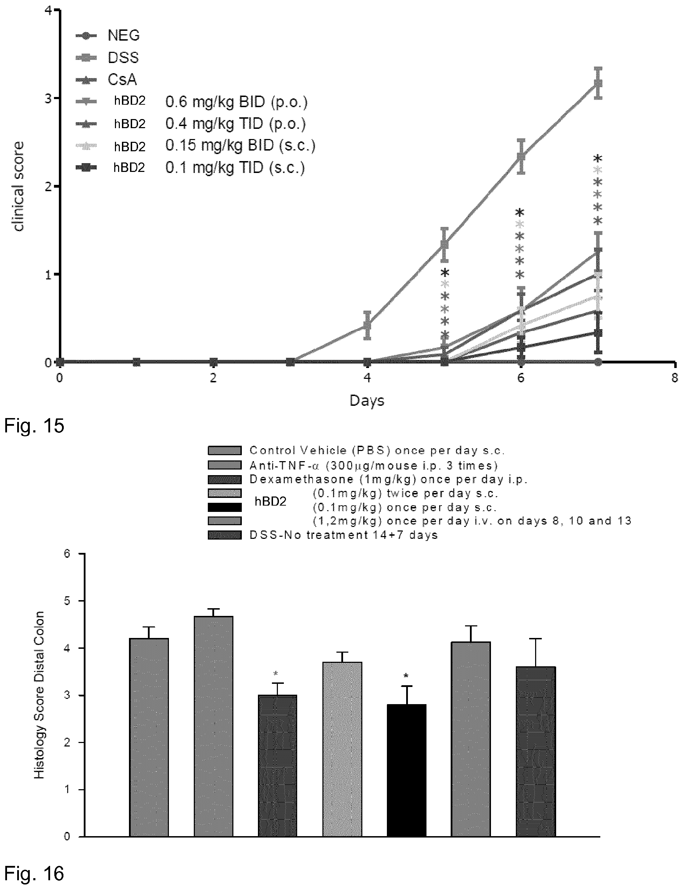

Figure 15 (for illustration). Clinical score (Stool score: 0=normal; 1=moist/sticky stool; 2=soft stool; 3=diarrhoea; Stool blood score: 0=no blood; 1=evidence of blood in stool or around anus; 2=severe bleeding; Mouse appearance: 0=normal; 1=ruffled fur or altered posture; 2=lethargic) in a murine 7-day prophylactic dextran sodium sulphate induced colitis study showing statistical significant effect of hBD2 on par with cyclosporine as well as superiority of TID dosing of hBD2 over BID dosing irrespective of dosing route. * p<0.05 vs. DSS; Two-way ANOVA and Bonferroni multiple comparisons test.
Figure 16 (for illustration). Histological score in a murine 14-day therapeutic dextran sodium sulphate induced colitis study showing statistical significant effect of hBD2 0.1 mg/kg on par with dexamethasone 1 mg/kg and superior to mouse anti TNF-α 300µg/mouse.

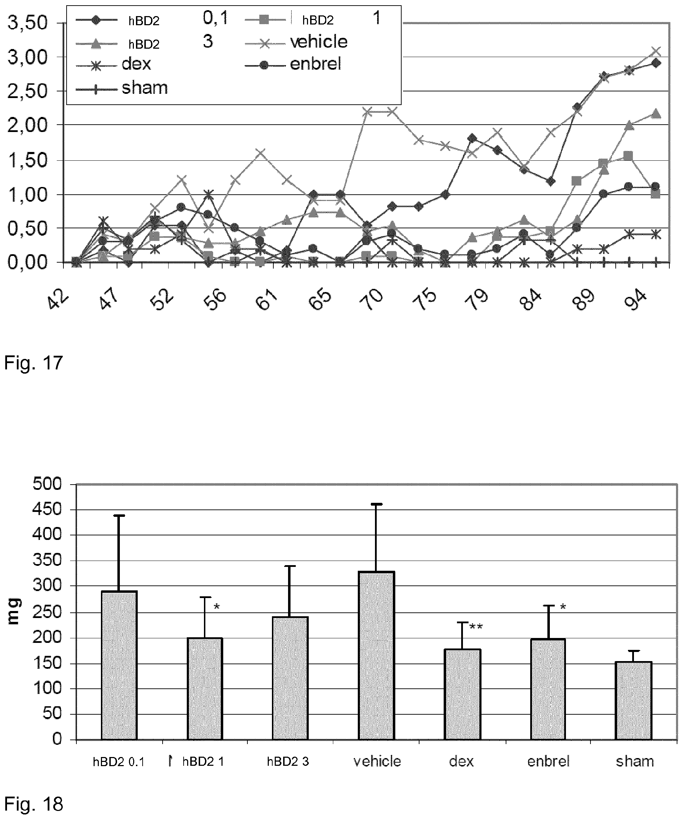

Figure 17 (for illustration). Clinical score (body weight loss, stool consistency and presence of blood per rectum) in a murine 14-week therapeutic SCID CD4+CD25+ Tcell transfer colitis model showing effect of hBD2 1 mg/kg s.c. OD on par with 100 µg/mouse anti TNF-α s.c. twice weekly (Enbrel) and 0.3 mg/kg dexamethasone intraperitoneally OD.
Figure 18 (for illustration). Colon weight in a murine 14-week therapeutic SCID CD4+CD25+ Tcell transfer colitis model showing effect of hBD2 1 mg/kg s.c. OD on par with 100 µg/mouse anti TNF-α s.c. twice weekly (Enbrel) and 0.3 mg/kg dexamethasone intraperitoneally OD. *p<0.05; **p<0.01 vs vehicle by t-test.

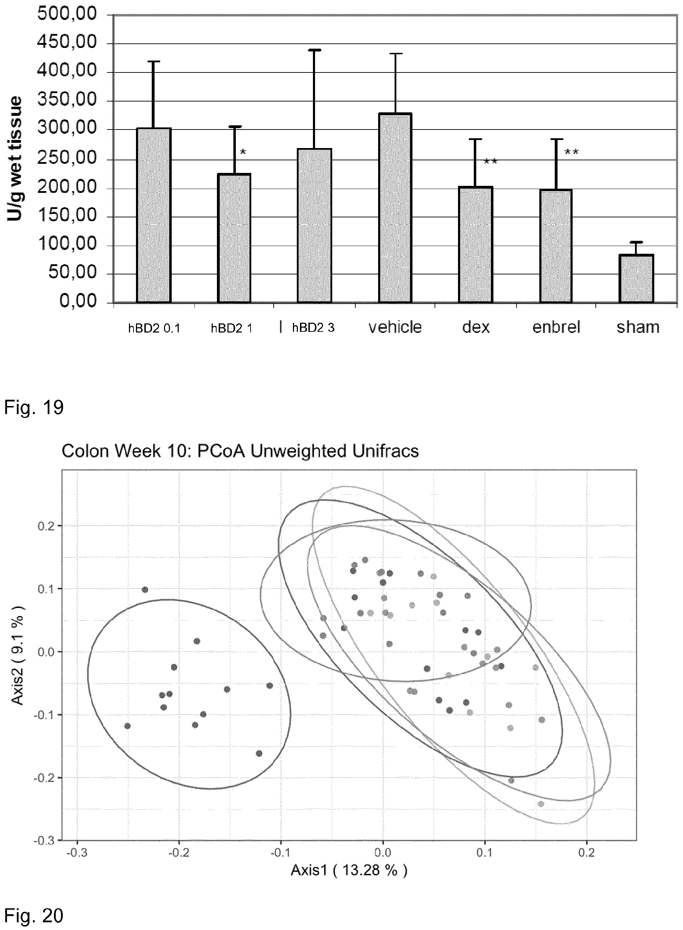

Figure 19 (for illustration). Myeloperoxidase activity in a murine 14-week therapeutic SCID CD4+CD25+ Tcell transfer model showing effect of 1 mg/kg s.c. hBD2 on par with 100 µg/mouse anti TNF-α s.c. twice weekly (Enbrel) and 0.3 mg/kg dexamethasone intraperitoneally OD. *p<0.05; **p<0.01 vs vehicle by t-test.

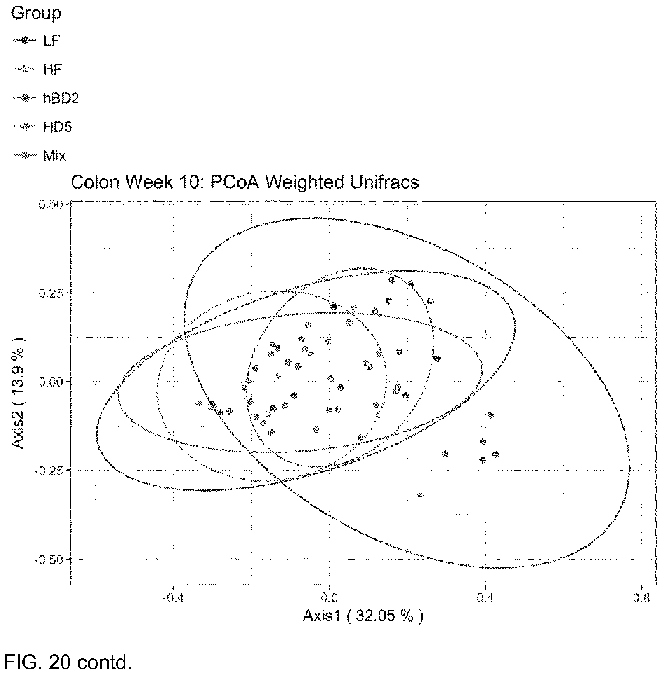

Figure 20 (for illustration). Unweighted and weighted unifrac analysis of microbial presence and abundance following prophylactic treatment with oral HD5, hBD2 and HD5+hBD2 in a murine high fat diet model.

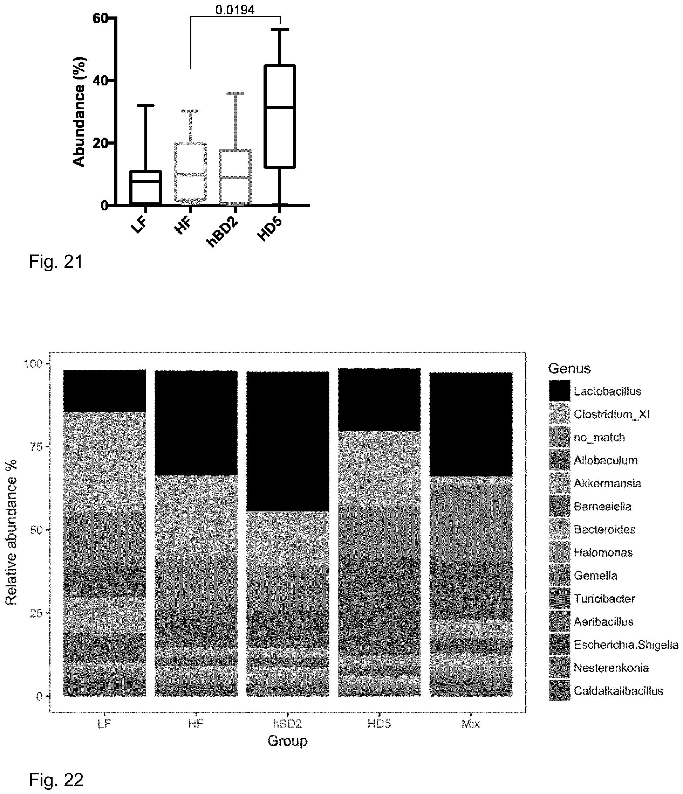

Figure 21 (for illustration). Abundance of Allobaculum in the small intestine following prophylactic treatment with oral HD5 and hBD2 in a murine high fat diet model.
Figure 22 (for illustration). Genus analysis of microbial abundance following prophylactic treatment with oral HD5, hBD2 and HD5+hBD2 in a murine high fat diet model.

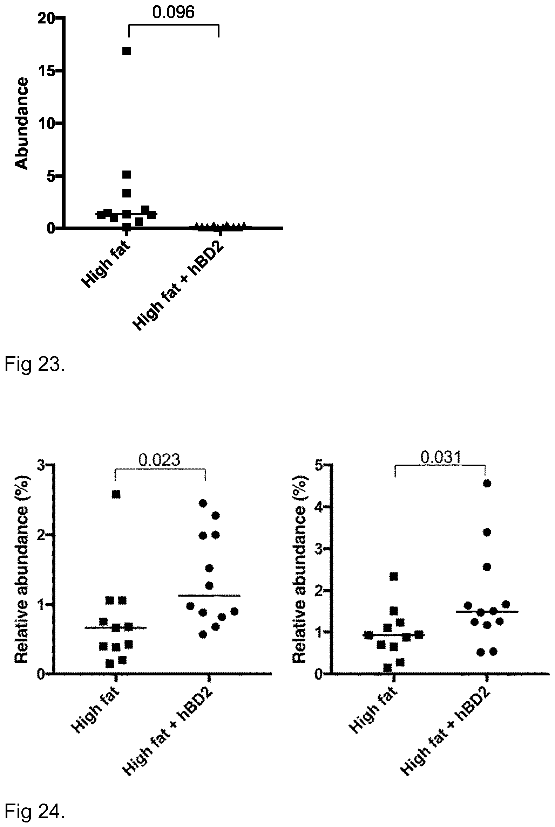

Figure 23 (for illustration). Abundance of Lactobacillaceae in colon following prophylactic treatment with oral hBD2 in a murine high fat model.
Figure 24 (for illustration). Relative abundance of Barnesiella in colon following 4 (left panel) and 10 weeks (right panel) of prophylactic treatment with oral hBD2 in a murine high fat diet model.

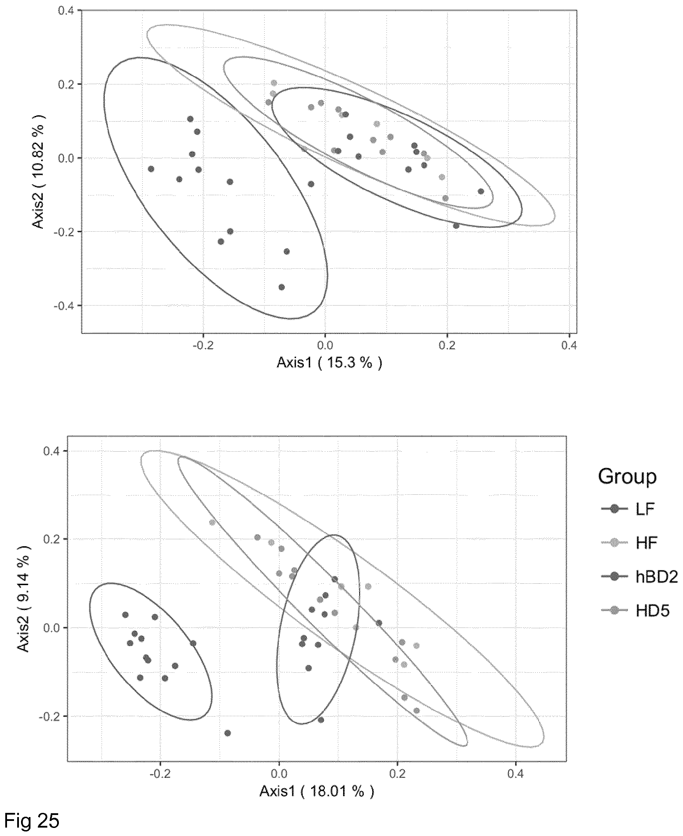

Figure 25 (for illustration). Unweighted unifrac analysis of microbial presence and abundance following therapeutic treatment with HD5 or hBD2 in a murine high fat diet model. Upper panel shows data at week 0. The lower panel at week 10.

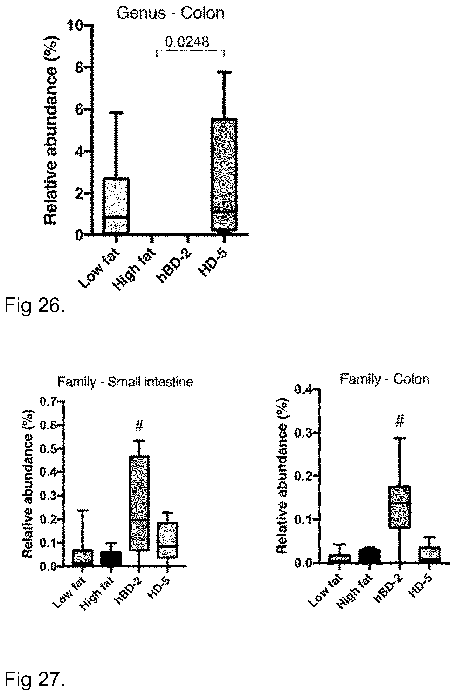

Figure 26 (for illustration). Relative abundance of Alloprevotella in colon following therapeutic intervention with oral HD5 and hBD2 in a murine high fat diet model.
Figure 27 (for illustration). Relative abundance of Bifidobacteriaceae in the small intestine and colon following therapeutic intervention with HD5 or hBD2 in a murine high fat diet model.
Figure legend for figures 28-31. Saline IN is the unchallenged and untreated control. HDM/Vehicle represent the untreated but HDM challenged animals. HDM are the animals challenged with house dust mites. PO is peroral administration and IN is intranasal administration. Columns labelled * are statistically significantly different from the vehicle treated control.
Figure 28 (for illustration): Airway hyper responsiveness in the murine House Dust Mite steroid-sensitive asthma model following prophylactic intranasal and oral administration of hBD2 respectively.
Figure 29 (for illustration): Pulmonary compliance in the murine House Dust Mite steroid-sensitive asthma model following prophylactic intranasal and oral administration of hBD2 respectively.

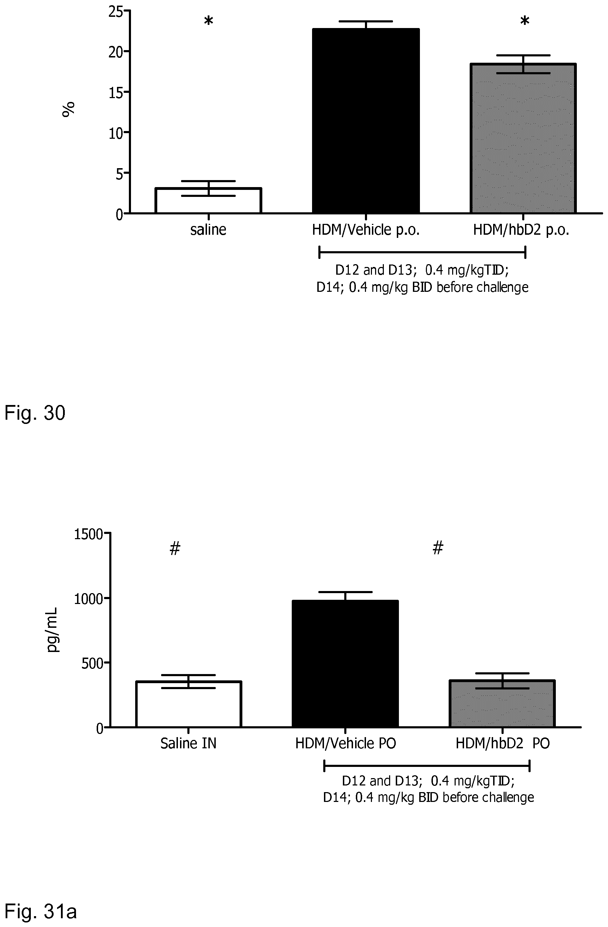

Figure 30 (for illustration): Neutrophil cell count in BALF in the House Dust Mite murine steroid-sensitive asthma model following prophylactic oral administration of hBD-2. The results are shown as mean +/- SEM.

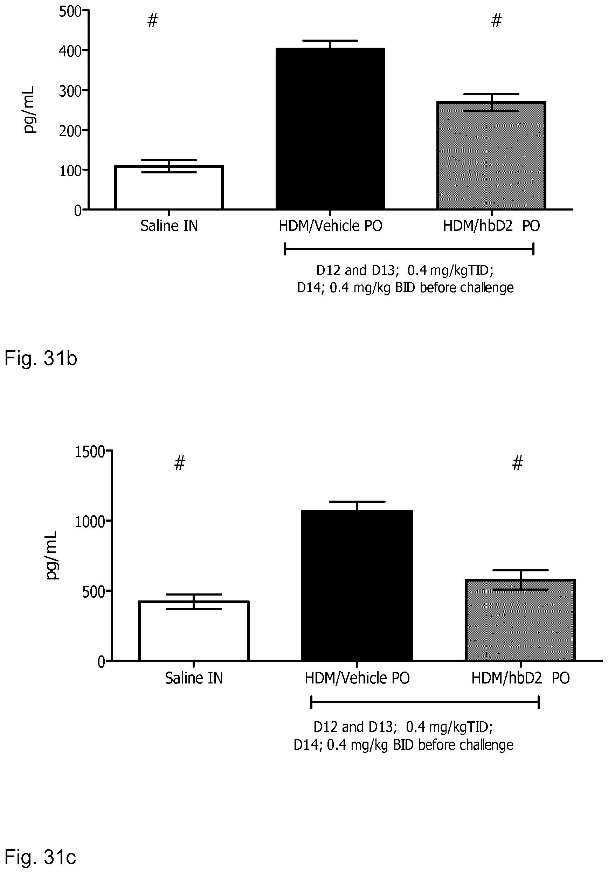

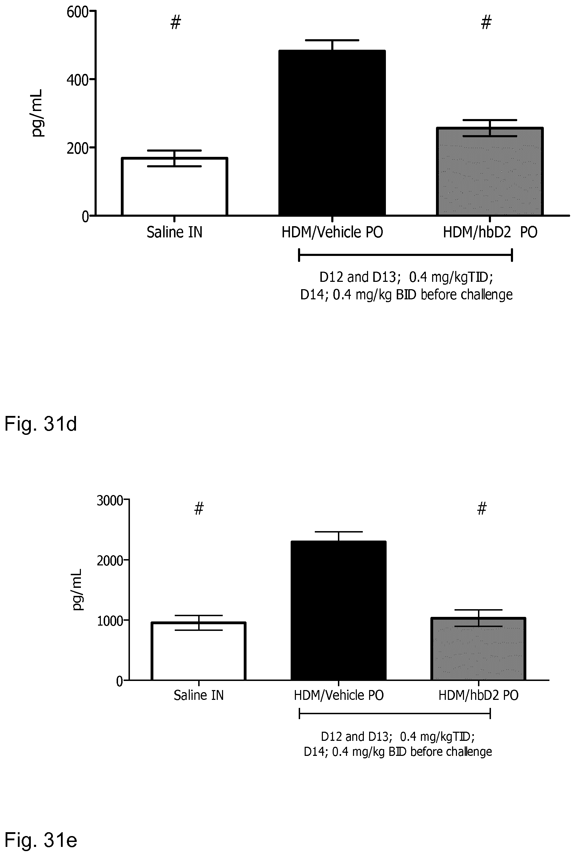

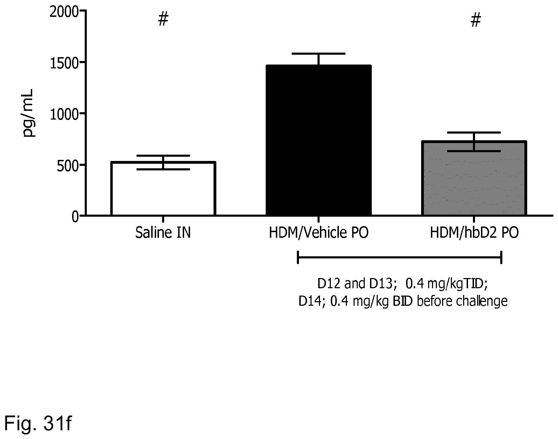

Figure 31a-f (for illustration). Cytokine concentrations of TNF-α (31a), IL-4 (31b), IL-5 (31c), IL-6 (31d), IL-9 (31e) and IL-13 (31f) in lung homogenate in the House Dust Mite murine steroid-sensitive asthma model following prophylactic oral administration of hBD-2. # p<0.05 vs HDM/vehicle PO, Mann Whitney test. The results are shown as mean +/- SEM.

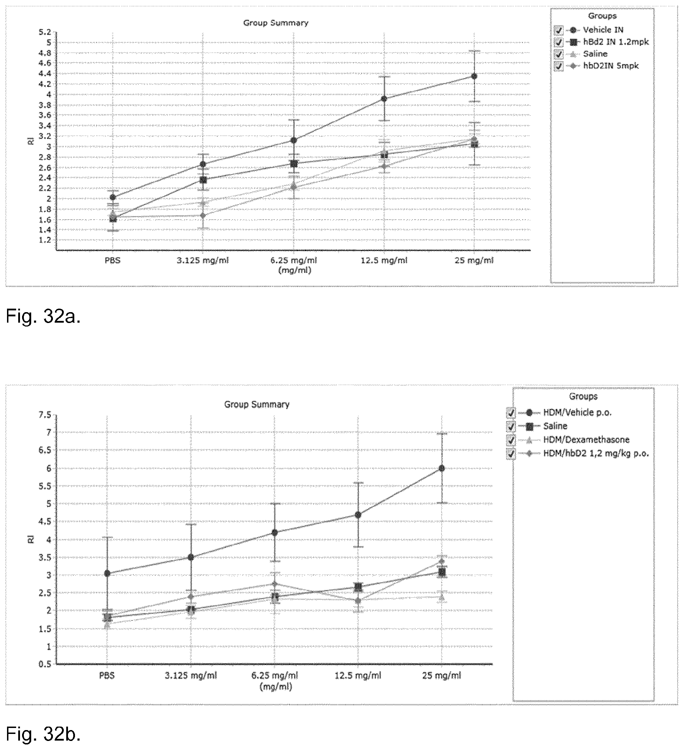

Figure 32a and 32b (for illustration): Airway hyper responsiveness in the House Dust Mite murine steroid-sensitive asthma model following therapeutic intranasal (figure 32a) and oral (figure 32b) administration of hBD2 respectively. Saline is the non-challenged control. HDM/Vehicle is the House Dust Mite challenged control treated with vehicle.
   "hBD2 IN 1.2 mpk" is hBD2 administered intranasally at 1.2 mg/kg. 5mpk is 5 mg/kg.

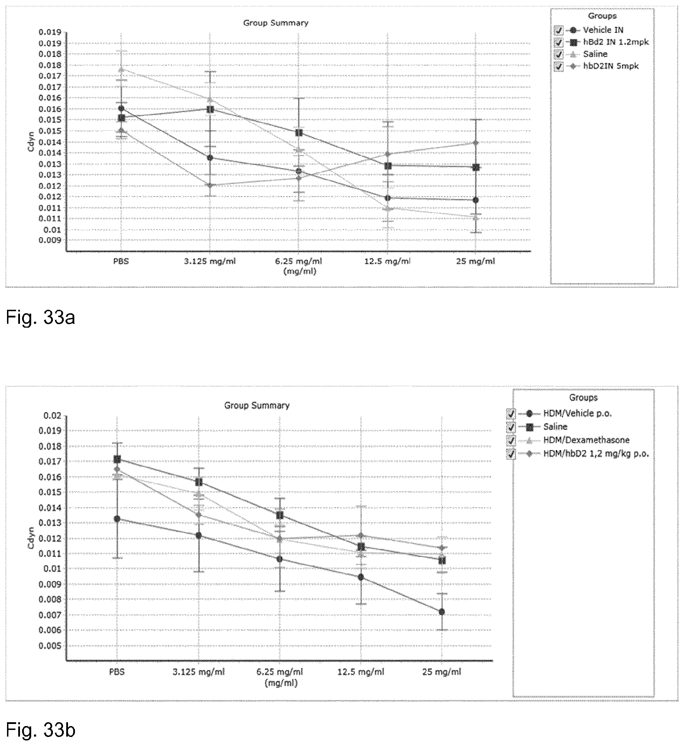

Figure 33a and 33b (for illustration): Pulmonary compliance in the House Dust Mite murine steroid-sensitive asthma model following therapeutic intranasal (figure 33a) and oral (figure 33b) administration of hBD2 respectively.

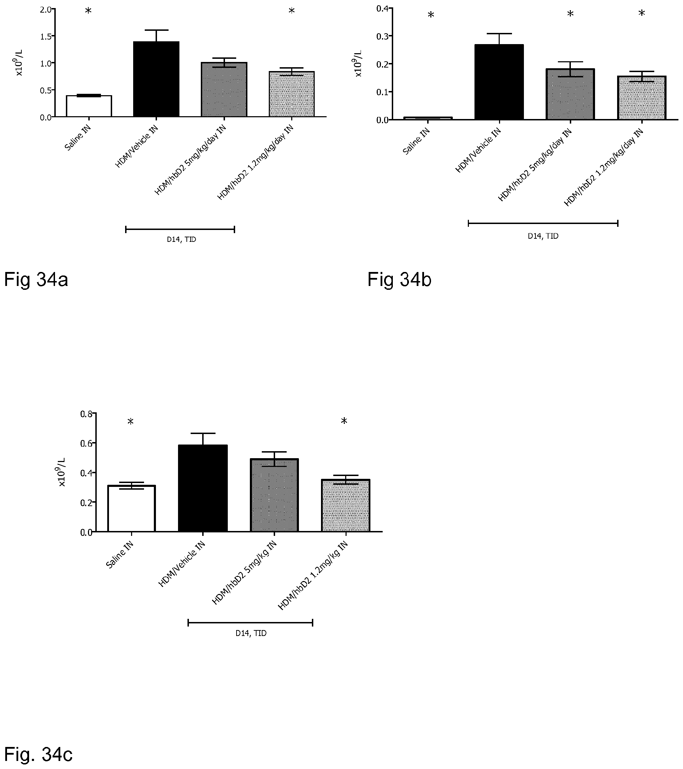

Figure 34 (for illustration): Total (34a), neutrophil (34b) and macrophage cell count (34c) in BALF in the House Dust Mite murine steroid-sensitive asthma model following therapeutic intranasal administration of hBD-2. *p<0.05 vs vehicle; Mann-Whitney test. The results are shown as mean +/- SEM.

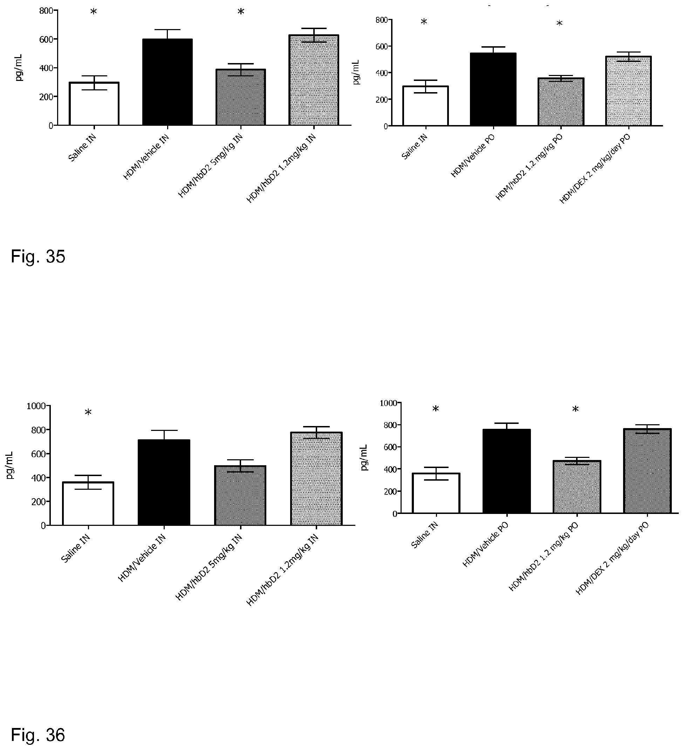

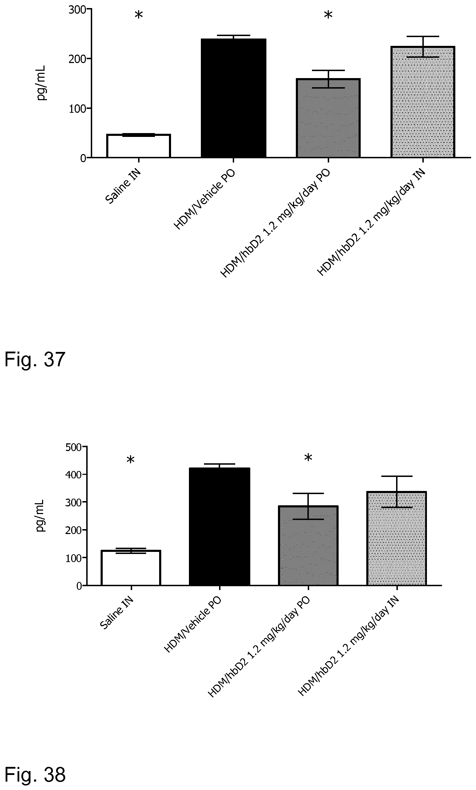

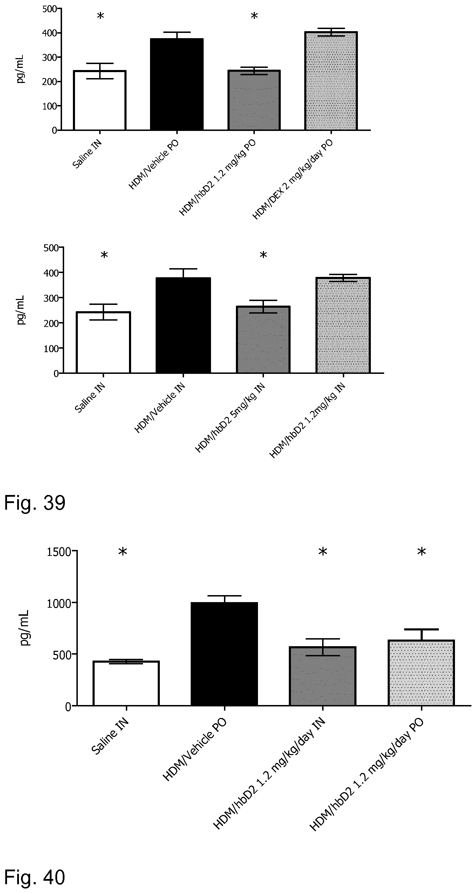

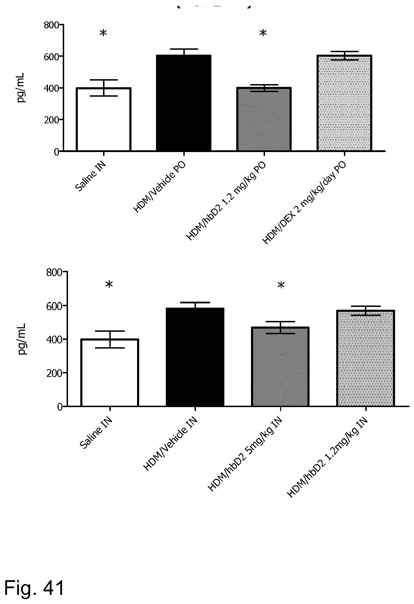

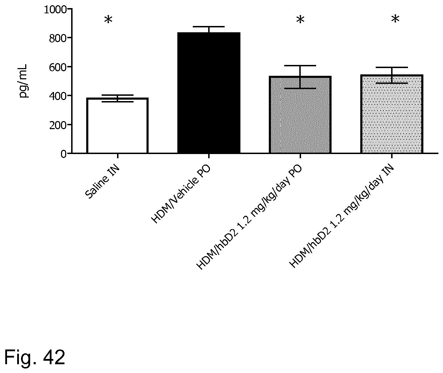

Figure 35-42 (for illustration). Cytokine concentrations of IFN-γ (Fig 35), TNF-α (Fig. 36), IL-4 (Fig. 37), IL-5 (Fig. 38), IL-6 (Fig. 39), IL-9 (Fig. 40), IL-10 (Fig. 41) and IL-13 (Fig. 42) in lung homogenate in the House Dust Mite murine steroid-sensitive asthma model following therapeutic intranasal and oral administration of hBD-2 respectively. Each figure has data from the intranasal arm on the left and the peroral arm on the right in those figures where both arm are shown. 'p<0.05 vs corresponding vehicle; Mann-Whitney test. The results are shown as mean +/- SEM.

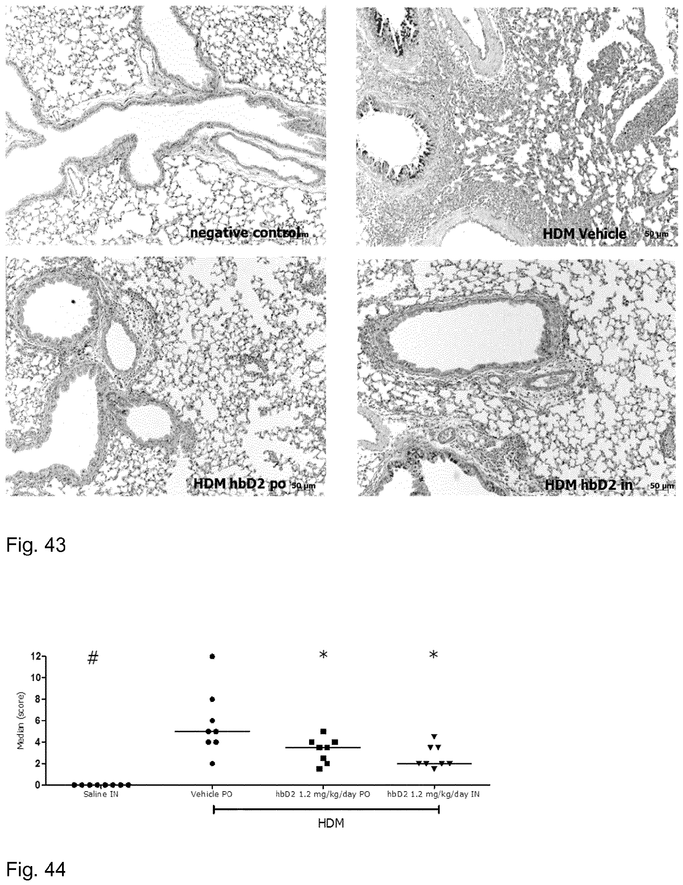

Figure 43 (for illustration). Lung histology with H&E/PAS preparation in the House Dust Mite murine steroid-sensitive asthma model following therapeutic intranasal and oral administration of hBD2 respectively. Upper left panel: untreated and unchallenged control. Upper right panel: untreated and HDM challenged control. Lower left panel: HDM challenged treated with hBD2 PO. Lower right panel: HDM challenged treated with hBD2 IN. 50X enlargement.
Figure 44 (for illustration). Lung inflammation severity in the House Dust Mite murine steroid-sensitive asthma model following therapeutic intranasal and oral administration of hBD2 respectively. *p<0.05 vs vehicle, Mann Whitney test; #p<0.05 vs vehicle, Wilcox Signed Rank Test.

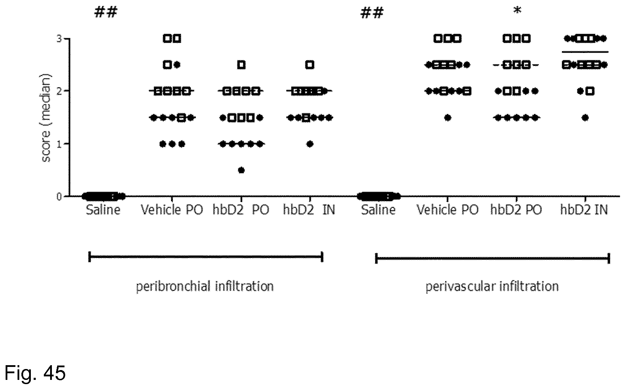

Figure 45 (for illustration). Perivascular and peribronchial inflammation in the House Dust Mite murine steroid-sensitive asthma model following therapeutic intranasal and oral administration of hBD2 respectively. *p<0.05 vs vehicle for perivascular infiltration of eosinophils, Mann Whitney test; #p<0.05 vs vehicle for perifaxcular/peribronchial infiltration of eosinophils and monocytes, Wilcox Signed Rank Test. □ monocytes; ◆ eosinophils.

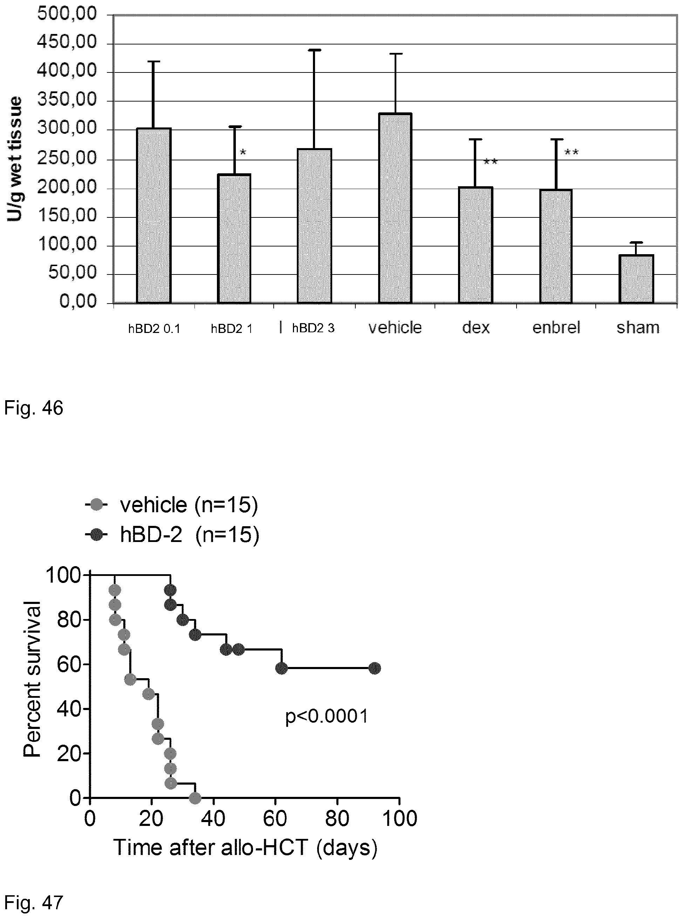

Figure 46 (for illustration). Myeloperoxidase activity in a murine 14-week therapeutic SCID CD4+CD25+ Tcell transfer model showing effect of 1 mg/kg s.c. hBD2 on par with 100 µg/mouse anti TNF-α s.c. twice weekly (Enbrel) and 0.3 mg/kg dexamethasone intraperitoneally OD. *p<0.05; **p<0.01 vs vehicle by t-test.
Figure 47. Kaplan-Meyer plot showing dramatically increased survival (p<0.0001) in a murine graft-versus-host disease model following stem cell transplantation at day 0 and treatment with hBD2 of 1.2 mg/kg/day or vehicle (PBS 100 µL/day) from day 0 to day 10 (n=15)
Figure 48.
   A statistically significant reduction of histology score of the small intestine, colon and liver in 10 mice treated with oral hBD-2 for 10 days from the day of stem cell transplantation.
Figure 49.
   Weight loss in % from baseline (a) and in gram (b) in mice treated with oral hBD-2 for 10 days from the day of stem cell transplantation.
Figure 50.
   Reduced CD45+ leucocyte cell migration into lamina propria of colon and small intestine in 10 mice treated with oral hBD-2 for 10 days from the day of stem cell transplantation.
Figure 51 a-c.
   Reduction of intestinal T cell and myeloid cell infiltration of the lamina propria of the colon and small intestine in 10 mice treated with oral hBD-2 for 10 days from the day of stem cell transplantation.
Figure 52 a-c.
   Cytokine concentrations of TNF-α (a), IL-6 (b) and IL-10 (c) in serum of 10 mice treated with oral hBD-2 for 10 days from the day of stem cell transplantation.
Figure 53 a-c.
   Reduced IL-1β production in myeloid cells in 10 mice treated with oral hBD-2 for 10 days from the day of stem cell transplantation.
Figure 54 a-f.
   Reduced proportion of neutrophils (a) and reduced Th 1 cytokine production - IFN-γ in CD4 T cells (b) and CD8 T cells (c) and CD69+CD4 T cells (d); TNF-α in CD4 T cells (e) and CD8 T cells (f) in 10 mice treated with oral hBD-2 for 10 days from the day of stem cell transplantation.
Figure 55.
   Increased inflammation, myeloid cell and leucocyte infiltration as well as increased tissue repair in colon in 10 mice treated with oral PBS for 10 days from the day of stem cell transplantation.
Figure 56.
   A statistically significant reduction of mortality (p = 0.03) in 7 mice treated with oral hBD-2 for 10 days from the day of stem cell transplantation compared with 13 mice treated with cyclosporine and PBS respectively.
Figure 57.
   Weight loss in % from baseline in mice treated with oral hBD-2, cyclosporine or PBS for 10 days from the day of stem cell transplantation.
Figure 58.
   A significant reduction of mortality in 22 mice treated with oral hBD-2, oral HD5 (for illustration) or PBS respectively for 10 days from the day of stem cell transplantation.

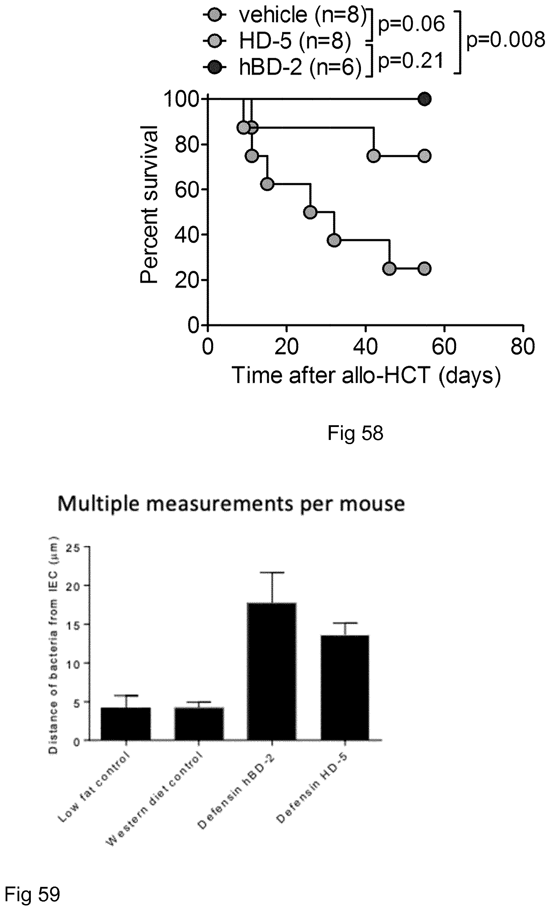

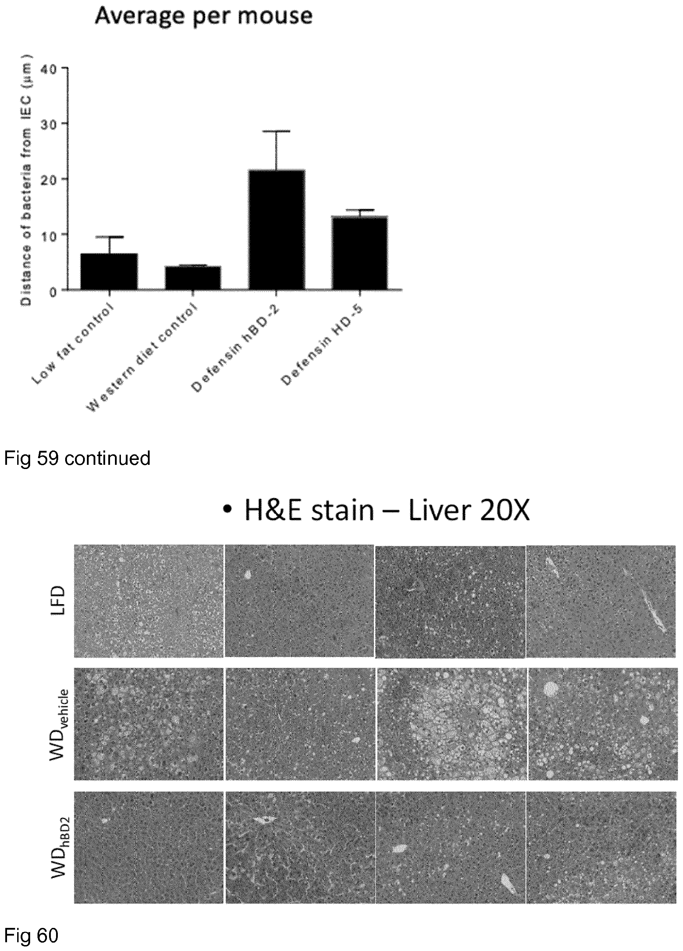

Figure 59 (for illustration).
   Bacterial population control exerted by orally administered HD5 and hBD-2 measured as the distance from the intestinal lining to bacterial population (lysis zone) in µm in mice compared with mice on a low fat or western diet. This experiment demonstrates that orally administered human HD5 and hBD-2 in a mouse surprisingly exerts their microbiota modulating effect at the epithelial surface as if they were mice defensins produced by the epithelial cells of the mouse itself and not primarily in the lumen of the intestine as one would expect following oral administration.
Figure 60 (for illustration).
   Hematoxylin/Eosin stain of liver following prophylactic treatment with hBD2 in a murine high fat diet model. No fat accumulation in the hepatocytes is noted in the mice on low fat diet whereas extensive steatosis (fat accumulation in hepatocytes) is noted in mice fed a western high fat diet and minimal intra hepatocyte fat accumulation is noted in the group of mice fed a western high fat diet supplemented by daily prophylactic administration of oral 1.2 mg/kg hBD-2.

### Detailed description

### Definitions:

Defensin: The term "defensin" as used herein refers to polypeptides belonging to the defensin class of antimicrobial peptides. Defensins represent one of the dominant innate host defences that serve to maintain a healthy microbiome and ward off potential pathogens (Wehkamp et al.et al., 2002 and Salzman et al., 2007). Defensins are peptides possessing antimicrobial activity against Gram positive and negative bacteria, fungi and archaea as well as exerting anti-inflammatory activity.

Human defensins are small cationic peptides divided into α- and β-defensins based on the topology of their three intramolecular cysteine disulphide bonds (Figure 6). Mammalian defensins have well-defined structural features: the mature peptides are small (3-6 kDa, 28-42 amino acids length), cationic (net charge +1 to +11), amphipathic and have a highly conserved tertiary structure of a triple antiparallel stranded β-sheet. The conservation of the structure amongst the defensins is somewhat surprising considering the variability in the amino acid sequence and is due to six conserved cysteine residues throughout the defensin family. These six cysteine residues form three disulphide bonds, which stabilize the triple-stranded β-sheet core structure (Mammalian Antimicrobial Peptides; Defensins and Cathelicidins Dorin et al, in Molecular Medical Microbiology (Second Edition), 2015.

α-defensins are those expressed by Paneth cells in the crypts of the small intestine (HD5 and HD6 or DEFA5 and DEFA6). β-defensins (DEFBn) are mainly produced by epithelial cells in various tissues and organs including the skin, eye, middle ear, mouth, trachea, lungs, gastrointestinal tract, urogenital system, kidneys, vagina, liver, pancreas and mammary glands. Examples of defensins include human intestinal alpha defensin 5 (HD5; SEQ ID NO: 5); human intestinal alpha defensin 6 (HD6; SEQ ID NO: 6); both belonging to the alfa defensin class; and also human beta defensin 1 (hBD1; SEQ ID NO: 1); human beta defensin 2 (hBD2; SEQ ID NO: 2); human beta defensin 3 (hBD3; SEQ ID NO: 3); human beta defensin 4 (hBD4; SEQ ID NO: 4); truncated human beta defensin 2 (SEQ ID NO: 7). Defensins are expressed as precursors and are processed by cleavage of the signal peptide and in some cases pro-peptides as well before secretion into the extracellular space. Some of the human defensins e.g. hBD1 are produced constitutively, whereas others e.g. hBD2, hBD3 and hBD4 are induced by pro-inflammatory cytokines or exogenous microbial products. The above-identified sequences represent the predicted mature bioactive defensins. It will be understood by one of skill in the art that processing may differ from cell to cell and that the resulting secreted mature peptide may differ by one or two C- or N-terminal amino acids from the predicted sequences and still retain bioactivity.

Identity: The relatedness between two amino acid sequences or between two nucleotide sequences is described by the parameter "identity". The degree of identity between two amino acid sequences is determined using the Needleman-Wunsch algorithm (Needleman and Wunsch, 1970, J. Mol. Biol. 48: 443-453) as implemented in the Needle program of the EMBOSS package (Rice *et* al.,2000, http://emboss.org), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the nobrief option) is used as the percent identity and is calculated as follows: (Identical Residues x 100)/(Length of Alignment - Total Number of Gaps in Alignment).

Normal microbiota: The term "normal microbiota" is used herein to indicate a microbiota that is not dysbiotic. Normal microbiota is characterized by having large gene richness. Normal intestinal microbiota is characterized by comprising bacteria belonging to the genera *Bacteriodetes, Faecalibacterium, Roseburia, Blautia, Ruminococcus, Coprococcus, Bifidobacterium, Methanobrevibacter, Lactobacillus, Coprococcus, Clostridium, Akkermansia, Eubacterium.*

Normal lung microbiota is characterized by comprising bacteria belonging to the genera *Bacteroidetes, Firmicutes,* and *Proteobacteria* with the core microbiota consisting of *Pseudomonas, Streptococcus, Prevotella, Fusobacteria, Veillonella, Haemophilus, Neisseria* and *Porphyromonas*

Treatment: The terms "treatment" and "treating" as used herein refer to the management and care of a patient for the purpose of combating a condition, disease or disorder. The term is intended to include the full spectrum of treatments for a given condition from which the patient is suffering, such as administration of the active compound for the purpose of: alleviating or relieving symptoms or complications; delaying the progression of the condition, disease or disorder; curing or eliminating the condition, disease or disorder; and/or preventing the condition, disease or disorder, wherein "preventing" or "prevention" is to be understood to refer to the management and care of a patient for the purpose of hindering, reducing the active compounds to prevent or reduce the risk of the onset of symptoms or complications. The patient to be treated is preferably a mammalian, in particular a human being.

Patient: A patient is a subject, which has been treated with or is about to receive an allogeneic hematopoietic stem cell transplantation.

### Mammalian alfa defensins and mammalian beta defensins.

This disclosure relates a human β-defensin selected from hBD-2 (SEQ ID NO.: 2), truncated hBD-2 (SEQ ID NO.: 7) or a functionally equivalent modified defensin comprising 1-4 amino acid modifications compared to hBD-2 of SEQ ID NO.: 2 or truncated hBD-2 of SEQ ID NO.: 7; wherein said modification(s) is a conservative amino acid substitution(s) or insertion(s) that do not significantly affect the folding of the defensin; or a single deletion(s), for use in the prevention or treatment of acute graft-versus-host-disease (GVHD) in a patient that has received or is about to receive an allogeneic hematopoietic stem cell transplantation.

In another embodiment, a defensin differs from one of the SEQ ID NO: 2, or 7 by less than 5, such as less than 4, for example less than 3, such as less than 2 amino acids. In a preferred embodiment, the defensins consist of human beta defensin 2 (SEQ ID NO: 2) and, truncated human beta defensin 2 (SEQ ID NO: 7).

In a preferred embodiment, the human beta defensins consists of human beta defensin 2 (SEQ ID NO: 2).

Preferably, the human β-defensins consist of human beta defensin 2, truncated human beta defensin 2, and functionally equivalent variants thereof as described in claim 1. More preferably, the human β-defensins consist of human beta defensin 2, and functionally equivalent variants as described in claim 1.

A "functionally equivalent variant" of a mammalian (e.g. human) alfa or beta defensin is a modified mammalian (e.g. human) alfa or beta defensin exhibiting approximatively the same effect on microbiota in the lung or the intestine or the skin as the parent mammalian (e.g. human) alfa and/or beta defensins. A functionally equivalent variant of a mammalian (e.g. human) defensin may comprise preferably 1-4 amino acid modifications, more preferably 1-3 amino acid modifications, most preferably 1-2 amino acid modification(s), and in particular one amino acid modification, as compared to the mammalian (e.g. human) defensin amino acid sequence (e.g. any of SEQ ID NO 1-6). Preferably, for beta mammalian defensins, compared to human beta defensin 2, having SEQ ID NO 2 and for alpha defensins compared to HD5 (SEQ ID NO 5).

The term "modification" of a human β-defensin for use according to claim 1 meanspreferably, amino acid modifications are of a minor nature, that is conservative amino acid substitutions or insertions that do not significantly affect the folding and/or activity of the polypeptide; single deletions; small amino- or carboxyl-terminal extensions; or a small extension that facilitates purification by changing net charge or another function, such as a poly-histidine tag, an antigenic epitope or a binding domain. In one embodiment the small extension, such as a poly-histidine tag, an antigenic epitope or a binding domain is attached to the mammalian (e.g. human) alfa or beta defensin through a small linker peptide of up to about 20-25 residues and said linker may contain a restriction enzyme cleavage site.

The Clustal W alignments in Figure 6 can be used to predict which amino acid residues can be substituted without substantially affecting the biological activity of the protein. The sequences were aligned using Clustal W 2.1 (http://www.geno,me.ip/tools/clustalw/ ) and the following settings: Gap Open Penalty: 10, Gap Extension Penalty: 0,05, Weight Transition: NO, Hydrophilic Residues for Proteins: GPSNDQE, Hydrophilic Gaps: YES, Weight Matrix: BLOSUM (for PROTEIN). Substitutions within the following group (Clustal W, 'strong' conservation group) are to be regarded as conservative substitutions:
-S,T,A; N,E,Q,K; N,H,Q,K; N,D,E,Q; Q,H,R,K; M,I,L,V; M,I,L,F; H,Y; F,Y,W. Substitutions within the following group (Clustal W, 'weak' conservation group) are to be regarded as semi-conservative substitutions: -C,S,A; A,T,V; S,A,G; S,T,N,K; S,T,P,A; S,G,N,D; S,N,D,E,Q,K; N,D,E,Q,H,K; N,E,Q,H,R,K; V,L,I,M; H,F,Y.

Examples of conservative substitutions are substitutions made within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions which do not generally alter specific activity are known in the art. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/lle, Leu/Val, Ala/Glu, and Asp/Gly.

"Unnatural amino acids" have been modified after protein synthesis, and/or have a chemical structure in their side chain(s) different from that of the standard amino acids. Unnatural amino acids can be chemically synthesized, and preferably, are commercially available, and include pipecolic acid, thiazolidine carboxylic acid, dehydroproline, 3- and 4-methylproline, and 3,3-dimethylproline.

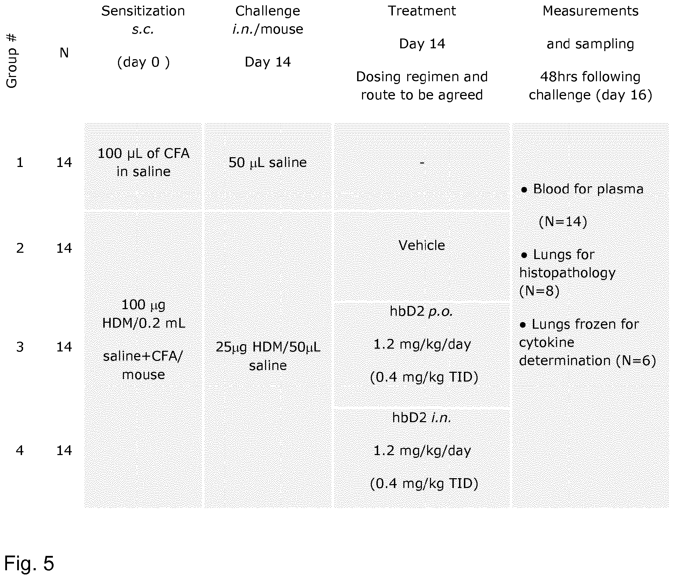

Essential amino acids in a mammalian alfa and/or beta defensin can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for biological activity *(i.e.,* activity against an airway hyper responsiveness or suppression cytokines e.g. TNF-alpha activity) to identify amino acid residues that are critical to the activity of the molecule. See also, Hilton et al., 1996, J. Biol. Chem. 271: 4699-4708. The identities of essential amino acids can also be inferred from analysis of identities with polypeptides which are related to mammalian alfa and/or beta defensins (see Clustal W alignment in figure 5).

Single or multiple amino acid substitutions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.,* Lowman et al., 1991, Biochem. 30:10832-10837; U.S. Patent No. 5,223,409; WO 92/06204), and region-directed mutagenesis (Derbyshire et al., 1986, Gene 46:145; Ner et al., 1988, DNA 7:127). When the result of a given substitution cannot be predicted with certainty, the derivatives may be readily assayed according to the methods described herein above to determine the presence or absence of biological activity.

### Long-acting defensins

The half-life of a human β-defensin for use according to claim 1 may be extended by fusing or conjugating the defensin with another moiety i.e. constructing a long acting biologically active defensin linked to a pharmaceutically acceptable molecule providing an *in vivo* plasma half-life of the defensin, which is increased substantially compared to the *in vivo* plasma half-life of the defensin administered in the same manner as the defensin.

A long acting biologically active - human β-defensin according to claim 1 comprising the defensin linked to a pharmaceutically acceptable molecule selected from the group consisting of a molecule having binding to a neonatal Fc receptor (FcRn), transferrin, albumin (HAS), XTEN^{®} or PEG, a homo-amino acid polymer (HAP), a proline-alanine-serine polymer (PAS), or an elastin-like peptide (ELP), hyaluronic acid, a negatively charged highly siasylated peptide such as the carboxy-terminal peptide (CTP) of chorionic gonadotropin (CG) β-chain, human IgG, and CH3(CH2)ₙCO- wherein n is 8 to 22.

The human β-defensin may be linked to the pharmaceutically acceptable molecule in various ways as described in the prior art literature, such as without limitation chemical coupling through a bifunctional linker, gene technologically by coupling the N-terminal or C-terminal of the defensin, such as α-defensin or β-defensin, to the pharmaceutically acceptable molecule, such as albumin or an albumin analogue. In particular, the N-terminal of albumin or an albumin analogue, e.g. human albumin, can be coupled to the C-terminal of the defensin, or the N-terminal of the defensin; or the C-terminal of albumin, e.g. human albumin, can be coupled to the C-terminal of the defensin, or the N-terminal of the defensin. A linker sequence can be inserted between the albumin and the defensin chain.

The human β-defensin may be linked to the pharmaceutically acceptable molecule through a stable linker or a more labile linker. Several linkers are known in the art, including bifunctional PEG molecules (e.g. see Paige et.al Pharmaceutical Research, vol. 12, no. 12, 1995), hydrolysable linkers (Shechter et al. Bioconjugate Chem. 2005,16: 913- 920 and International Journal of Peptide Research and Therapeutics, Vol. 13, Nos. 1-2, June 2007 and WO2009095479), PDPH and EMCH see e.g. in WO2010092135. In the special case where chemical conjugation (linking of two or more molecules) of the defensin, to the pharmaceutically acceptable molecule, strongly reduce the functional defensin activity, it may be preferable to use a more labile linker that can release the functional defensin.

Half-life extension may also be accomplished through acylation of the peptide backbone with a spacer e.g. γ-L-glutamyl spacer and a C-18 fatty di-acid chain to Lysine. The fatty di-acid site chain and the spacer mediate a strong but reversible binding to albumin, slowing release from the injection site and reducing renal clearance.

### Methods and Uses

Human beta defensin-2 and HD5 have been tested in both preventive and therapeutic animal models of severe intestinal inflammation and dysbiosis (Dextran sodium sulphate, Trinitrobenzene sulfonic acid and CD4+CD25+ T-cell transfer). α- and β-defensins whether administered orally or subcutaneously mitigate diarrhoea and weight loss induced by these chemicals through improved intestinal health. Importantly the defensins decrease the disease activity index and myeloperoxidase activity as a marker of inflammation on par with strong standard GVHD immunosuppressants such as prednisolone/dexamethasone and cyclosporin as well as anti TNF-α, all routinely used in the treatment of GVHD. The defensins thus show potent activity as a prophylactic and/or therapeutic treatment of patients that have had or are about to have an allogeneic hematopoietic stem cell transplantation.

It has been demonstrated that hBD-2 can rebalance the immune system through normalization of IFN-γ, TNF-α, IL-4, IL-5, IL-6, IL-9, IL-10 and IL-13 tissue concentrations thus preventing or treating the cytokine storm leading to acute GVHD and thus shown potent activity as a prophylactic and/or therapeutic treatment of patients that have had or are about to receive an allogeneic hematopoietic stem cell transplantation.

It has been demonstrated in both a preventive and a therapeutic high fat diet mouse model that both HD5 and hBD2 have a strong influence on the composition of the microbiota increasing the variety of microbes i.e. preserve/normalize/reinstall the microbiota as well as promote the abundance of key commensal bacteria, thus presenting a method for treating dysbiosis, a common feature of acute GVHD.

It has been demonstrated that hBD2 can normalize gut health, inflammation and function through a decrease of myeloperoxidase activity, thus presenting a method for improving gut health in patients that have had an allogeneic hematopoietic stem cell transplantation.

It has further been demonstrated in a mouse model of acute allergic asthma that a dosage of human beta-defensin 2 (hBD2) whether administered orally or intranasally is capable of preventing the development of asthma, thus presenting a method for prevention of lung complications and decreased lung capacity often associated with acute and chronic GVHD.

It has also been demonstrated in a mouse model of acute allergic asthma that a dosage of human beta-defensin 2 (hBD2) whether administered orally or intranasally is also capable of treating asthma, thus presenting a method for treating lung complications and reduced lung capacity often associated with acute and chronic GVHD.

In other aspects, the disclosure relates to a human β-defensin for use according to claim 1 in a method of treatment according to any of the methods described herein.

The provided human β-defensin for use according to claim 1 can treat or prevent lung and/or gut inflammation and/or liver inflammation by changing bacterial phenotypes through a change at the transcriptional level as well as structure and composition of the lung and/or gut and/or liver bacterial flora or the lung and/or gut metabolome in a subject that has been treated or is about to be treated with allogeneic hematopoietic stem cell transplantation.

Without being bound by theory the effects observed using oral administration may be ascribed to a change in the gut microflora and gut metabolome that may have an effect on the lungs through the so-called gut-lung axis. Chronic lung disorders such as asthma, COPD and chronic GVHD all exhibit a component of intestinal disease manifestation indicating that there is a vital cross talk between these two mucosal sites of the human body and a variety of respiratory diseases have been associated with a dysbiosis not only of the airway microbiota but also the intestinal microbiota.

Commensal microbes calibrate innate and adaptive immune responses and impact activation thresholds for pathogenic stimulations, in large part by producing small molecules that mediate host-microbial interactions (Donia and Fishback, 2015). While the epithelial barrier ensures that microorganisms are largely confined to the gut, microbial metabolites can penetrate the epithelial barrier, allowing them to enter and accumulate in the host circulatory system where they are sensed by immune cells (Dorrestein, 2014). Trompette, 2013 demonstrated in mice that fermentable fibers in the diet changed the composition not only of the gut but also the lung microbiota in particular the ratio of *Firmicutes* to *Bacteriodetes,* the latter leading to increased local and systemic levels of Short Chain Fatty Acids, which in turn influenced Dendritic Cell hematopoiesis and functionality thus shaping the immunological environment in the lung and influencing the severity of allergic inflammation. Schirmer et al (2016) further demonstrated in the Human Functional Genomics Project that inter-individual variation in cytokine response is linked to specific microbial organisms as well as microbial functions. The majority of detected associations were both cytokine and stimulus specific, suggesting that the immune system recognizes and interacts with microbial organisms and products with high specificity and that these microbial factors are associated with a particular immunological phenotype. TNF-α and IFN-γ production capacity appeared to be more strongly influenced by the microbiome, whereas other cytokines such as IL-1β, IL-6 and Th17 derived IL-17 and IL-22 exhibited fewer, but more specific, associations with the gut microbiota.

The human β-defensin for use according to claim 1 can be administered in combination with either prebiotics, probiotics, tryptophane, glucocorticoids, cyclosporine, anti TNF-α, integrins, antibiotics, immunosuppressants, fecal transplantation or a combination of these. The defensins can be administered separately or together with one or more of these therapies. The defensins can also be administered together with other medicaments which can be used in the treatment of patients that have had or are about to have an allogeneic hematopoietic stem cell transplantation.

Normalizing the gut and/or lung microbiota may also involve changing the metabolome to one that produces relatively more butyrate or tryptophane and relatively less acetate.

### In vitro synthesis

Mammalian antimicrobial peptides including mammalian alfa defensins and mammalian beta defensins may be prepared by *in vitro* synthesis, using conventional methods as known in the art. Various commercial synthetic apparatuses are available, for example automated synthesizers by Applied Biosystems Inc., Beckman, etc. By using synthesizers, naturally occurring amino acids may be substituted with unnatural amino acids, particularly D-isomers (or D-forms) e.g. D-alanine and D-isoleucine, diastereoisomers, side chains having different lengths or functionalities, and the like. The particular sequence and the manner of preparation will be determined by convenience, economics, purity required, and the like. Chemical linking may be provided to various peptides or proteins comprising convenient functionalities for bonding, such as amino groups for amide or substituted amine formation, e.g. reductive amination, thiol groups for thioether or disulphide formation, carboxyl groups for amide formation, and the like. If desired, various groups may be introduced into the peptide during synthesis or during expression, which allow for linking to other molecules or to a surface. Thuscysteines can be used to make thioethers, histidines for linking to a metal ion complex, carboxyl groups for forming amides or esters, amino groups for forming amides, and the like.

Mammalian antimicrobial peptides including mammalian alfa defensins, mammalian beta defensins or functional equivalents thereof, may also be isolated and purified in accordance with conventional methods of recombinant synthesis. Recombinant synthesis may be performed using appropriate expression vectors and a eukaryotic or prokaryotic expression system. A solution may be prepared of the expression host and the media and the defensins present purified using HPLC, exclusion chromatography, gel electrophoresis, affinity chromatography, or other purification technique. Methods for recombinant expression of human beta defensin-2 in E. coli are disclosed in WO 2010/007166 (Novozymes).

The mammalian alfa and beta defensins, may also be induced by administration of the corresponding mRNA.

### Dosages

The human β-defensin for use according to claim 1 is preferably employed in an amount which is effective to prevent or treat graft-versus-host-disease in a patient that have had a allogeneic hematopoietic stem cell transplantion preferably with acceptable toxicity to the patient. A human β-defensin for use according to claim 1is also preferably employed in an amount which is effective to maintain a normal microbiota composition in the lung and/or the intestine or to treat or normalize a dysbiotic microbiota in the lung and/or the intestine, preferably with acceptable toxicity to the patient in need of the treatment.

For such treatments, the appropriate dosage will, of course, vary depending upon, for example, the chemical nature and the pharmacokinetic data of a compound used, the individual host, the mode of administration and the nature and severity of the conditions being treated.

In one embodiment an indicated daily dosage of a human β-defensin for use according to claim 1 is preferably from about 0.1 mg hBD2 /kg body weight to about 10 mg hBD2 /kg body weight, more preferably from about 0.5 mg hBD2 /kg body weight to about 10 mg hBD2 /kg body weight; such as 1 mg hBD2 /kg body weight to 10 mg hBD2 /kg body weight, more preferably from about 1.2 mg hBD2 /kg body weight to about 10 mg hBD2 /kg body weight, preferably from about 1.2 mg hBD2 /kg body weight to about 5 mg hBD2 /kg body weight, even more preferably 1.2 mg hBD2 /kg body weight, for example, administered in divided doses up to one, two or three times a day.

When two different defensins are administered in one dosage, the dosage may comprise equal or approximately equal amounts of the two defensins determined on a weight basis or on a molar basis. The ratio may also differ so that the ratio of alpha defensin to the human beta-defensin varies from 10:1 to 1:10, such as 5:1 to 1:5, for example 2:1 to 1:2 determined on a weight or molar basis.

The compounds of preferred embodiments can be administered to mammals, for example humans, by similar modes of administration at similar dosages than conventionally used.

In one embodiment, the daily dosage of the defensin for use according to claim 1 is between 0.1 and 10 mg defensin/kg, such as between 0.5 and 5 mg defensin/kg, such as between 1 and 2 mg defensin/kg, such as 1.2 mg defensin/kg per day.

In certain embodiments, the human beta defensin for the use of claim 1 can be used in an amount of about 0.1 mg or less to about 1500 mg or more per unit dosage form, preferably from about 0.1, 0.2, 0.3, 0.4, or 0.5 mg to about 150, 200, 250, 300, 350, 400, 450, 500, 600, 700, 800, 900, or 1000 mg, and more preferably from about 1, 2, 3, 4, 5, 6, 7, 8, 9, 10, 15, 20, or 25 mg to about 30, 35, 40, 45, 50, 55, 60, 65, 70, 75, 80, 85, 90, 95, or 100 mg. In certain embodiments, however, lower or higher dosages than those mentioned above may be preferred. Appropriate concentrations and dosages can be readily determined by one skilled in the art..

In one embodiment, the human β-defensin for use according to claim 1 is administered at least once daily, such as at least twice daily, for example at least 3 times daily or continuously.

### Administration

In one embodiment, the human β-defensin for use according to claim 1 is administered to a patient for a period prior to the patient receiving allogeneic hematopoietic stem cell transplantation.

In this case the administration of defensin may be continued during the transplantation and may further be administered after the transplantation is complete. In one embodiment, the human β-defensin for use according to claim 1 is administered to a patient at the time of receiving allogeneic hematopoietic stem cell transplantation. For example, the defensin may be administered on the same date as the transplantation. The human β-defensin administration may be continued after transplantation.

In one embodiment, the human β-defensin for use according to claim 1 is administered after allogeneic hematopoietic stem cell transplantation. In this embodiment, administration of the defensin may be continued once allogeneic hematopoietic stem cell transplantation is completed. For example, the defensin may be administered on the same date as the transplantation and one or more times each subsequent day following transplantation. In one embodiment, the treatment continues after transplantation by administration at intervals such as every day, every second day, every third day, every fourth day, every fifth day, every sixth day or every seventh day. As exemplified in example 13, the treatment may start on the day of the transplantation and be continued for days after the transplantation, such as 1 day or more, for example 2, 3, 4, 5, 6, 7, 8, 9, 10, 11, 12, 13, 14, 15, 16, 17, 18, 19 or 20 days after the transplantation. The treatment may also have been begun prior to the transplantation and continued for days after the transplantation, such as 1 day or more, for example 2, 3, 4, 5, 6, 7, 8, 9, 10, 11, 12, 13, 14, 15, 16, 17, 18, 19 or 20 days after the transplantation.

### Formulations for oral or parenteral administration

The human β-defensins for use according to claim 1 can be formulated for administration by any conventional route.

In one embodiment, the administration is oral, buccal, sublingual, rectal, vaginal, intratracheal, intrapulmonary, intranasal, intracranial, subcutaneous, intravenous, dermal or transdermal. Preferably the administration is oral.

In one embodiment, the administration of the human β-defensin for use according to claim 1, is oral.

In one embodiment, the administration of the human β-defensin for use according to claim 1, is generally intranasal or intrapulmonary. Intranasal and intrapulmonary administration is normal for pulmonary drug delivery.

In one embodiment, the administration of the human β-defensin for use according to claim 1, is subcutaneous or intravenous.

Within some embodiments the human β-defensin for use according to claim 1 may be formulated as a lyophilizate, utilizing appropriate excipients that provide stability as a lyophilizate, and subsequent to rehydration. In a preferred embodiment, the human β-defensin for use according to claim 1 is formulated as a sterile and isotonic solution. Pharmaceutically acceptable carriers and/or diluents are familiar to those skilled in the art. For human β-defensins for use according to claim 1 formulated as liquid solutions, acceptable carriers and/or diluents include saline and sterile water should be included, and the composition may optionally include antioxidants, buffers, bacteriostats, and other common additives.

The disclosed human β-defensin for use according to claim 1may be formulated in a wide variety of formulations for oral administration. Solid form preparations may include powders, tablets, drops, capsules, cachets, lozenges, and dispersible granules. Other forms suitable for oral administration may include liquid form preparations including emulsions, syrups, elixirs, aqueous solutions, aqueous suspensions, toothpaste, gel dentifrice, chewing gum, or solid form preparations which are intended to be converted shortly before use to liquid form preparations, such as solutions, suspensions, and emulsions.

The disclosed human β-defensin for use according to claim 1 may be formulated in a wide variety of formulations for buccal, sublingual, oral, rectal, vaginal, dermal, transdermal, intracranial, subcutaneous or intravenous administration. The formulation can contain (in addition to a mammalian alfa defensin and/or a mammalian beta defensin and other optional active ingredients) carriers, fillers, disintegrators, flow conditioners, sugars and sweeteners, fragrances, preservatives, stabilizers, wetting agents, emulsifiers, solubilizers, salts for regulating osmotic pressure, buffers, diluents, dispersing and surface-active agents, binders, lubricants, and/or other pharmaceutical excipients as are known in the art. One skilled in this art may further formulate the human β-defensin in an appropriate manner, and in accordance with accepted practices, such as those described in Remington's Pharmaceutical Sciences, Gennaro (1990).

The human β-defensin for use according to claim 1, can be used alone, or in combination therapies with one, two, or more other pharmaceutical compounds or drug substances, for example with prebiotics, probiotics, glucocorticoids, cyclosporine, anti TNF-α, integrins, antibiotics, immunosuppressants or a combination of these and/or with one or more pharmaceutically acceptable excipient(s).

### Airway Administration

Airway administration may be used for administering the human β-defensin for use according to claim 1 of the invention. By intrapulmonary administration is meant topical administration to the lungs. When used herein the terms "intratracheal, intrabronchial or intra alveolar administration" include all forms of such administration whereby a defensin is applied into the trachea, the bronchi or the alveoli, respectively, whether by instillation of a solution of a defensin, by applying a defensin in a powder form, or by allowing a defensin to reach the relevant part of the airway by inhalation of a defensin as an aerosolized or nebulized solution or suspension or inhaled powder or gel, with or without added stabilizers or other excipients.

Methods of intrabronchial/alveolar administration include, but are not limited to, bronchoalveolar lavage (BAL) according to methods well known to those skilled in the art, using as a lavage fluid a physiologically acceptable composition in which the human β-defensinfor use according to claim 1 has been dissolved or indeed by any other effective form of intrabronchial administration including the use of inhaled powders containing defensins in dry form, with or without excipients, or the direct application of a defensin, in solution or suspension or powder form during bronchoscopy. Methods for intratracheal administration include, but are not limited to, blind tracheal washing with a similar solution of dissolved defensins or a defensin suspension, or the inhalation of nebulized fluid droplets containing dissolved defensins or a defensin suspension obtained by use of any nebulizing apparatus adequate for this purpose.

In another embodiment, intratracheal, intrabronchial or intra alveolar administration does not include inhalation of the product but the instillation or application of a solution of a defensin or a powder or a gel containing defensin into the trachea or lower airways.

Other preferred methods of administration may include using the following devices:
1. Pressurized nebulizers using compressed air/oxygen mixture
2. Ultrasonic nebulizers
3. Electronic micropump nebulizers
4. Metered dose inhaler (MDI)
5. Dry powder inhaler systems (DPI),

The aerosol may be delivered via a) facemasks or b) via endotracheal tubes in intubated patients during mechanical ventilation (device 1, 2 and 3). The devices 4 and 5 can also be used by the patient without assistance provided that the patient is able to self-activate the aerosol device.

Preferred concentrations for the human β-defensin for use according to claim 1, where said defensin is in a solution are in the range of from about 0.1 µg to 1000 µg per ml solution, such as in the range of from about 0.1 µg to 250 µg per ml solution.

### Pharmaceutical composition for intrapulmonary administration

The human β-defensin for use according to claim 1, may be comprised in a formulation in combination with, preferably dissolved in, a pharmaceutically acceptable carrier, preferably an aqueous carrier or diluent, or carried to the lower airways as a pegylated preparation or as a liposomal or nanoparticle preparation administered as an aerosol via inhalation, or as a lavage fluid administered via a bronchoscope as a bronchoalveloar lavage or as a blind intratracheal wash or lavage. A variety of aqueous carriers may be used, including, but not limited to 0.9% saline, buffered saline, physiologically compatible buffers and the like.. The resulting aqueous solutions may be packaged for use or filtered under aseptic conditions and freeze-dried, the freeze-dried preparation being dissolved in a sterile aqueous solution prior to administration.

In one embodiment a freeze-dried defensin preparation may be pre-packaged for example in single dose units. In an even more preferred embodiment the single dose unit is adjusted to the patient.

The human β-defensin for the use according to claim 1 may be combined with pharmaceutically acceptable auxiliary substances or adjuvants, including, without limitation, pH adjusting and buffering agents and/or tonicity adjusting agents, such as, for example, sodium acetate, sodium lactate, sodium chloride, potassium chloride, calcium chloride, etc.

The human β-defensin for use according to claim 1 may be formulated with pharmaceutically acceptable carriers and excipients including microspheres, liposomes, microcapsules, nanoparticles or the like. Conventional liposomes are typically composed of phospholipids (neutral or negatively charged) and/or cholesterol. The liposomes are vesicular structures based on lipid bilayers surrounding aqueous compartments. They can vary in their physiochemical properties such as size, lipid composition, surface charge and number and fluidity of the phospholipids bilayers. The most frequently used lipid for liposome formation are: 1,2-Dilauroyl-*sn*-Glycero-3-Phosphocholine (DLPC), 1,2-Dimyristoyl-*sn*-Glycero-3-Phosphocholine (DMPC), 1,2-Dipalmitoyl-sn-Glycero-3-Phosphocholine (DPPC), 1,2-Distearoyl-*sn*-Glycero-3-Phosphocholine (DSPC), 1,2-Dioleoyl-*sn*-Glycero-3-Phosphocholine (DOPC), 1,2-Dimyristoyl-*sn*-Glycero-3-Phosphoethanolamine (DMPE), 1,2-Dipalmitoyl-*sn*-Glycero-3-Phosphoethanolamine (DPPE), 1,2-Dioleoyl-*sn*-Glycero-3-Phosphoethanolamine (DOPE), 1,2-Dimyristoyl-*sn*-Glycero-3-Phosphate (Monosodium Salt) (DMPA), 1,2-Dipalmitoyl-*sn*-Glycero-3-Phosphate (Monosodium Salt) (DPPA), 1,2-Dioleoyl-*sn-*Glycero-3-Phosphate (Monosodium Salt) (DOPA), 1,2-Dimyristoyl-*sn*-Glycero-3-[Phospho-rac-(1-glycerol)] (Sodium Salt) (DMPG), 1,2-Dipalmitoyl-*sn*-Glycero-3-[Phospho-rac-(1-glycerol)] (Sodium Salt) (DPPG), 1,2-Dioleoyl-*sn*-Glycero-3-[Phospho-rac-(1-glycerol)] (Sodium Salt) (DOPG), 1,2-Dimyristoyl-*sn*-Glycero-3-[Phospho-L-Serine] (Sodium Salt) (DMPS), 1,2-Dipalmitoyl-*sn*-Glycero-3-[Phospho-L-Serine) (Sodium Salt) (DPPS), 1,2-Dioleoyl-*sn*-Glycero-3-[Phospho-L-Serine] (Sodium Salt) (DOPS), 1,2-Dioleoyl-*sn*-Glycero-3-Phosphoethanolamine-N-(glutaryl) (Sodium Salt) and 1,1',2,2'-Tetramyristoyl Cardiolipin (Ammonium Salt). Formulations composed of DPPC in combination with other lipids or modifiers of liposomes are preferred e.g. in combination with cholesterol and/or phosphatidylcholine.

Long-circulating liposomes are characterized by their ability to extravasate at body sites where the permeability of the vascular wall is increased. The most popular way of producing long-circulating liposomes is to attach hydrophilic polymer polyethylene glycol (PEG) covalently to the outer surface of the liposome. Some of the preferred lipids are: 1,2-Dipalmitoyl-*sn*-Glycero-3-Phosphoethanolamine-N-[Methoxy(Polyethylene glycol)-2000] (Ammonium Salt), 1,2-Dipalmitoyl-*sn*-Glycero-3-Phosphoethanolamine-N-[Methoxy(Polyethylene glycol)-5000] (Ammonium Salt), 1,2-Dioleoyl-3-Trimethylammonium-Propane (Chloride Salt) (DOTAP).

Possible lipids applicable for liposomes are supplied by Avanti, Polar Lipids, Inc, Alabaster, AL. Additionally, the liposome suspension may include lipid-protective agents which protect lipids against free-radical and lipid-peroxidative damage on storage. Lipophilic free-radical quenchers, such as alpha-tocopherol and water-soluble iron-specific chelators, such as ferrioxianine, are preferred.

A variety of methods are available for preparing liposomes, as described in, e.g., Szoka et al., Ann. Rev. Biophys. Bioeng. 9:467 (1980), U.S. Pat. Nos. 4, 235,871, 4,501,728 and 4,837,028. Another method produces multilamellar vesicles of heterogeneous sizes. In this method, the vesicle-forming lipids are dissolved in a suitable organic solvent or solvent system and dried under vacuum or an inert gas to form a thin lipid film. If desired, the film may be redissolved in a suitable solvent, such as tertiary butanol, and then lyophilized to form a more homogeneous lipid mixture which is in a more easily hydrated powder-like form. This film is covered with an aqueous solution of the targeted drug and the targeting component and allowed to hydrate, typically over a 15-60 minute period with agitation. The size distribution of the resulting multilamellar vesicles can be shifted toward smaller sizes by hydrating the lipids under more vigorous agitation conditions or by adding solubilizing detergents such as deoxycholate.

Micelles are formed by surfactants (molecules that contain a hydrophobic portion and one or more ionic or otherwise strongly hydrophilic groups) in aqueous solution.

Common surfactants well known to one of skill in the art can be used in the micelles of the present invention. Suitable surfactants include sodium laureate, sodium oleate, sodium lauryl sulfate, octaoxyethylene glycol monododecyl ether, octoxynol 9 and PLURONIC F-127 (Wyandotte Chemicals Corp.). Preferred surfactants are nonionic polyoxyethylene and polyoxypropylene detergents compatible with IV injection such as, TWEEN-80, PLURONIC F-68, n-octyl-beta-D-glucopyranoside, and the like. In addition, phospholipids, such as those described for use in the production of liposomes, may also be used for micelle formation.

### Examples

**Example 1** (for illustration).

Anti-inflammatory effect of s.c. and i.v. administration of hBD2 versus intraperitoneal mouse anti TNF-α in a prophylactic, murine, 10-day, oral Dextran Sodium Sulphate induced colitis model.

### Treatment regime:

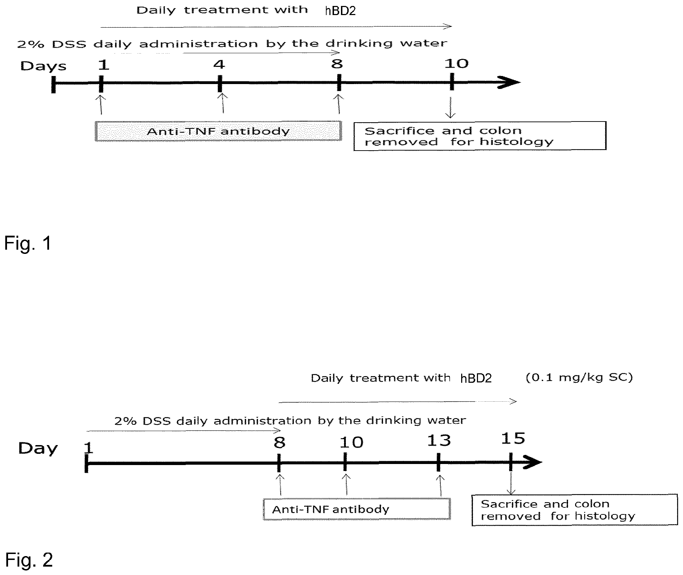

Figure 1 illustrates the study design.

70 male C57BL/6 mice housed in groups of five per cage were allocated to 7 different treatment groups. All animals had their drinking water supplemented with DSS 2% for 7 days to induce colitis resulting in significant inflammation and injury of the colonic tissue. The animals were treated with hBD2 dosed intravenously in the tail vein or subcutaneously under the dorsal skin, with mouse anti TNF-α intraperitoneally or PBS as vehicle for 10 days.

### Tests:

A disease activity index (DAI) scoring system was applied for clinical assessment (Figure 7). The animals were sacrificed on day 10 and colon removed for histological assessment employing a histological scoring system (Figure 8).

### Results:

The most significant effect was obtained when hBD2 was administered via the subcutaneous route, either once or twice daily, but intravenous administration of hBD2 also resulted in significant reduction of DAI. Anti TNFα and hBD2 both showed a similar and statistically significant effect on DAI. hBD2 especially when administered s.c. twice daily had a highly statistical significant effect on histology score whereas anti TNF-α showed no significant effect on this score (Figure 9).

Acute GVHD is characterized by crypt cell necrosis and in severe cases total loss of epithelium throughout the intestines. The chemically induced murine DSS model, which causes loss of epithelium thus seems relevant to test the prophylactic effect of compounds in GVHD. The results in example 1 demonstrate that especially s.c. administration of hBD2 is as effective as anti TNF-α treatment. Some publications have reported a positive effect of anti-TNF -α in GVHD.

**Example** 2 (for illustration).

Anti-inflammatory effect of oral administration of hBD2 versus oral prednisolone in a prophylactic murine, 10-day, oral Dextran Sodium Sulphate induced colitis model.

### Treatment regime:

50 male C57BL/6 mice housed in groups of five per cage were allocated to 5 different treatment groups. All animals had their drinking water supplemented with DSS 2% for 7 days to induce colitis resulting in significant inflammation and injury of the colonic tissue. The animals were treated with oral hBD2 dosed at three different dose levels twice per day or prednisolone 1 mg/kg twice per day or PBS as vehicle for 10 days.

### Tests:

A disease activity index (DAI) scoring system was applied for clinical assessment (Figure 7). The animals were sacrificed on day 10 and colon removed for histological assessment employing a histological scoring system (Figure 8).

### Results:

A dramatically reduced loos of body weight indicating that these animals were eating more and experienced a better health status was seen for all three hBD2 dose levels but most pronounced in the high dose group receiving 5 mg/kg BID (Figure 10). The two lower dose levels of hBD2 had a statistically significant effect on DAI on par with Prednisolone, whereas the high dose hBD2 (5 mg/kg BID) had a significantly better effect on DAI than these (Figure 11). Both Prednisolone and hBD2 showed a statistically significant effect on histological score but the effect of all three hBD2 dose groups were significantly better than Prednisolone (Figure 12).

Acute GVHD is characterized by crypt cell necrosis and in severe cases total loss of epithelium throughout the intestines. The chemically induced murine DSS model, which causes loss of epithelium thus seems relevant to test the prophylactic effect of compounds in GVHD. The results from example 2 demonstrate that oral hBD2 administration is as effective as the standard GVHD prophylactic treatment with oral prednisolone.

**Example 3** (for illustration).

Anti-inflammatory effect of s.c. administration of hBD2 versus s.c. prednisolone in a prophylactic murine, 8-day Trinitrobenzene Sulfonic Acid induced colitis model.

### Treatment regime:

105 male BALB/cByJ mice housed in groups of five per cage were allocated to 7 different treatment groups. Colitis was induced by intracolonic administration of trinitrobenzene sulfonic acid on day 0 under light anaesthesia resulting in significant inflammation and injury of the colonic tissue. The animals were treated with hBD2 at four different dosing levels once daily (1 mg/kg, 0.3 mg/kg, 0.1 mg/kg, and 0.03 mg/kg) or Prednisolone 10 mg/kg once daily dosed subcutaneously under the dorsal skin or PBS as vehicle for 7 days.

### Tests:

A disease activity index (DAI) scoring system was applied for clinical assessment (Figure 7). The animals were sacrificed on day 10 and colon removed for histological assessment employing a histological scoring system (Figure 8).

### Results:

A highly statistically significant increase in body weight indicating that these animals were eating more and experienced a better health status was seen for the two lowest (0.03 and 0.1 mg/kg OD) hBD2 dose levels. Both Prednisolone and hBD2 (at 0.1, 0.3 and 1 mg/kg) showed a highly statistically significant clinical effect on histological score (Figure 13).

Acute GVHD is characterized by crypt cell necrosis and in severe cases total loss of epithelium throughout the intestines. The chemically induced murine TNBS model, which causes a severe loss of epithelium thus seems relevant to test the prophylactic effect of compounds in GVHD. The results from example 3 demonstrate that s.c. administration of hBD2 is as effective as the standard preventive treatment with prednisolone.

**Example 4** (for illustration).

Anti-inflammatory effect of oral and s.c. administration of hBD2 versus oral cyclosporine in a prophylactic, murine, 7-day, Dextran Sodium Sulphate induced colitis model.

### Treatment regime:

80 male C57BL/6 mice housed in groups of five per cage were allocated to 7 different treatment groups. All animals had their drinking water supplemented with DSS 3% for 7 days to induce colitis characterized by epithelial damage, disruption of crypt architecture and a moderate inflammation characterized by accumulation of mononuclears and neutrophils in mucosa and submucosa. The animals were treated with oral hBD2 dosed at 0.6 mg/kg BID or 0.4 mg/kg TID; hBD2 dosed s.c. at 0.15 mg/kg BID or 0.1 mg/kg TID; or cyclosporine 50 mg/kg OD or water for 7 days.

### Tests:

Clinical assessment was conducted as per a scoring system (0=no blood in stool; 1=evidence of blood in stool and 2=severe bleeding). The animals were sacrificed on day 7 and colon removed for histological assessment.

### Results:

A highly statistically significant improvement in maintenance of colon length (Figure 14) and clinical scores (Figure 15) was observed for all four hBD2 dosing groups as well as for Cyclosporine. TID dosing of hBD2 was more efficacious than BID dosing with both administration routes and s.c. dosing seemed more efficacious than oral dosing (Figure 15).

Acute GVHD is characterized by crypt cell necrosis and in severe cases total loss of epithelium throughout the intestines. The chemically induced murine DSS model, which causes severe loss of epithelium thus seems relevant to test the prophylactic effect of compounds in GVHD. The results from example 4 demonstrate that both s.c. and oral hBD2 administration is as effective especially on clinical assessment as the standard GVDH prophylactic treatment with cyclosporine.

**Example 5** (for illustration).

Anti-inflammatory effect of s.c. administration of hBD2 versus intraperitoneal anti TNF-α and intraperitoneal dexamethasone in a therapeutic, murine, 14-day, Dextran Sodium Sulphate induced colitis model.

### Treatment regime:

70 male C57BL/6 mice housed in groups of five per cage were allocated to 7 different treatment groups. All animals had their drinking water supplemented with DSS 2% for 14 days to induce colitis resulting in significant inflammation and injury of the colonic tissue. The animals were treated with hBD2, 0.1 mg/kg s.c. BID; 0.1 mg/kg s.c. OD or 1.2 mg/kg i.v. OD from day 8 to day 14 or with mouse anti TNFα 250 µg/mouse intraperitoneally at day 8, 10 and 13 or with dexamethasone 1 mg/kg intraperitoneally OD or PBS s.c. as vehicle.

### Tests:

A disease activity index (DAI) scoring system was applied for clinical assessment (Figure 7). The animals were sacrificed on day 14 and colon removed for histological assessment employing a histological scoring system (Figure 8)

### Results:

A statistically significant effect on DAI was observed for hBD2 0.1 mg s.c. OD and for Dexamethasone 1 mg/kg i.p. OD. Similarly a statistically significant effect on histological score (Figure 16) was observed for both hBD2 0.1 mg/kg s.c. OD and Dexamethasone.

Acute GVHD is characterized by crypt cell necrosis and in severe cases total loss of epithelium throughout the intestines. The chemically induced murine DSS model, which causes severe loss of epithelium thus seems relevant to test the treatment effect of compounds in GVHD. The results from example 5 demonstrate that s.c. administration of hBD2 is as effective as the standard treatment of GVHD with dexamethasone.

Example 6 (for illustration).

Efficacy of s.c. administration of hBD2 versus s.c. anti TNF-α (Enbrel) and intraperitoneal dexamethasone in a therapeutic, murine, 14 weeks, CD4+CD25+ Tcell transfer induced colitis model.

### Treatment regime:

70 female BALB/c mice housed in groups of five per cage were allocated to 7 different treatment groups. Colitis was induced in SCID mice by transplantation of CD4+CD25+ Tcells from BALB/c mice. Lymphocytes isolated from spleen and lymph nodes from BALB/c mice were subjected to negative selection of CD4+ Tcells. Afterwards, CD4+CD25+ cells were positively isolated by binding to the beads from the CD4+ Tcell suspensions and the CD4+CD25- were collected from the supernatant. The animals were treated with hBD2, 0.1 mg/kg s.c. OD; 1 mg/kg s.c. OD or 3 mg/kg s.c. OD or with mouse anti TNFα 100 µg/mouse s.c. twice weekly or with dexamethasone 0.3 mg/kg intraperitoneally OD or PBS s.c. as vehicle from day 7 and 86 consecutive days.

### Tests:

Clinical assessment was based on body weight loss, stool consistency and presence of blood per rectum. The animals were sacrificed on day 95 and colon removed for assessment of weight and myeloperoxidase activity.

### Results:

A statistically significant and similar effect on clinical score was observed for hBD2 1 mg/kg s.c. OD, for Dexamethasone 0.3 mg/kg i.p. OD and for Enbrel (Figure 17). Similarly these three dosing regimens showed a statistically significant and similar effect on colon weight (Figure 18) and myeloperoxidase activity (Figure 19).

Acute GVHD is believed to be a T-cell mediated syndrome characterized by crypt cell necrosis and in severe cases total loss of epithelium throughout the intestines and especially the CD4+CD25+ T reg cells have received a lot of attention as part of the pathogenesis of acute GVHD (Ball & Egeler, 2008). The murine CD4+CD25+ T cell transfer model, which causes severe loss of epithelium thus seems relevant to test the treatment effect of compounds in severe and persistent GVHD.

Myeloperoxidase is a human enzyme, that plays an important role in initiation and progression of inflammation and is a biomarker of oxidative stress and inflammation and has recently been reported as a potential biomarker of GVHD (Ge and Ye, 2013). The results from example 6 demonstrate that hBD2 administration is as effective as the standard anti-inflammatory treatment of GVHD with dexamethasone but also the less established treatment with anti TNF-α.

**Example 7** (for illustration).

Protection and preservation of gut microbiota by prophylactic treatment with defensins. Mice: Mice were housed in trios, 4 cages per group. Feed intake was registered daily just before lights were turned off at 6 pm. Individual mice were subjected to experimental procedures in altered order both group and cage wise. Mice were kept at room temperature under a 12-hour light/dark cycle at SPF standard conditions.

Diets: For dosing, the average weight was estimated to be 25 grams per mouse. Mice eat approximately 3 grams of feed per mouse per day.

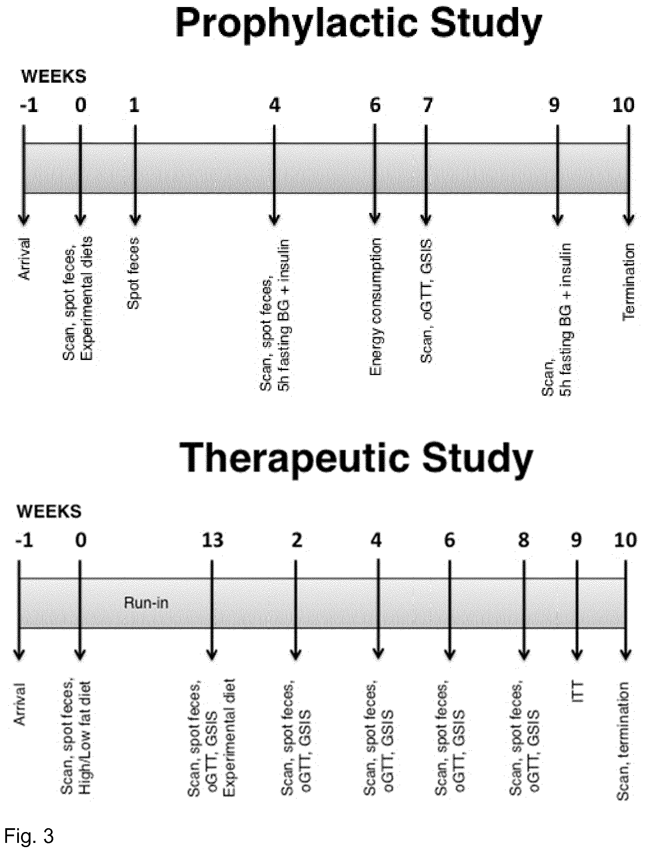

Treatment regime: Mice were fed either a high fat diet (HFD) or a low fat (LF) control diet. The HFD contained 4 subgroups; 1 hBD2, 1 HD5, 1 hBD2/HD5 and 1 standard HFD without supplementation of defensins (Figure 3). Defensin concentration was 1,2 mg hBD2 per kg mouse per day. HD5 was given in equimolar concentration to hBD2. The combinatory group was given 50% hBD2 + 50% HD5, hence a total amount of defensins equivalent to the remaining test groups.

### Tests:

Microbial analyses were carried out to study the microbiota of the intestine.

Longitudinal 16S characterization was conducted on 4 paired samples from 60 mice, 240 samples in total. Each mouse was sampled prior to diet change, 1 week post diet change, 4 weeks post diet change and at termination, thus ensuring a thorough characterization of the faecal microbiota as a result of defensin treatment.

### Results

Weight change. While the food intake was similar in all three experimental diet groups, both High Fat Diet (HFD) groups gained significantly more weight than the Low Fat Diet (LFD) reference group over the 10 week study period (*p<0.0001, 2-way ANOVA, Tukey Post Test). The HFD plus hBD2 group, however, gained significantly less weight than the HFD reference group (*p= 0.0028).

Microbiota. hBD2 affected primarily the microbial presence, whereas HD5 and hBD2+HD5 affected primarily the microbial abundance (Figure 20). A statistically significant increase of abundance of Allobaculum was seen in the small intestine following prophylaxis with HD5 (p<0.02; Figure 21). Allobaculum is a short chain fatty acid producing species. Short chain fatty acids play an important role in regulating colonic Treg cell homeostasis mediated via GPCR43. Tcell hypoplasia and perturbed Tcell homeostasis is a central part of both acute and chronic GVHD. Figure 22 is a genus analysis that shows the relative abundance of species in the different treatment groups and illustrate the effect of hBD2 and HD5 on intestinal flora. A statistically significant increase in abundance of Barnesiella in the colon was observed following prophylactic treatment with hBD2 (p<0.03; Figure 24). Barnesiella is a bacteria that is able to eliminate and protect against the intestinal dominance of antibiotic-resistant pathogenic bacteria that can be observed in hospitalized patients. The abundance of Barnesiella corresponds with the amount of several immunoregulatory cells. The higher the level of Barnesiella in the colon, the more marginal zone B cells and invariant natural killer T cells enumerated in the spleen and liver. In the development of colitis in IL-10-/- mice, higher levels of a Barnesiella phylotype correlated with lower activity levels of the disease. A trend towards lower abundance of Lactobacillaceae was observed in colon following prophylactic treatment with hBD2 (p=0.1; Figure 23).

The HFD group of mice developed extensive steatosis (intra hepatocytic fat accumulation) whereas the HFD group of mice treated with prophylactically with hBD-2 developed minimal steatosis (Figure 60).

Microbiota seems to play an important role in the development of GVHD especially following the dramatic bacterial decontamination carried out just after the stem cell transplantation. In particular, microbiota seems to play an important role in the development of GVHD especially following the dramatic bacterial decontamination carried out just prior to the stem cell transplantation. This example 7 demonstrates that defensins have a profound influence on the microbiota composition in terms of number of species present as well as overall number of bacteria and thus seem to protect and preserve a healthy microbiota. More specifically defensins seem to promote Short Chain Fatty Acid producing bacteria, SCFA that play a key role in colonic Treg cell homeostasis. In addition defensins seem to protect against liver inflammation and steatosis.

**Example 8** (for illustration). Treatment of dysbiosis by interventional treatment with defensins.

Mice and diets. The experiment elucidates the effect of hBD2 and HD5 on the microbiota in diet-induced obese mice. A run-in period of 13 weeks where mice were fed a very HFD (60% energy from fat) preceded the intervention. Only mice meeting the criteria of a minimum of 12 gram weight gain (approximately 50% of initial bodyweight) during the run-in period were included in the final analyses. Mice that did not meet these criteria stayed in their respective cages as hierarchy 'keepers'. They were exposed to all experimental tests, but excluded from the analyses.

Treatment regimen. Before the intervention all mice were MR scanned. Cages of mice were allocated to experimental groups based on their fat mass. All subsequent measures were paired with data from the same mouse before the intervention. A LFD (low fat diet) reference group was running in parallel. As controls for the intervention 2 additional groups were included: 1 very HFD and 1 LFD. Experimental mice stayed on the very HFD during the intervention (Figure 3). The mice were on the experimental diet for 10 weeks. They were co-housed throughout the experiment, 4 mice per cage, 3 cages per group. All tests ran over 3 days, 1 cage per group per day.

Tests. Microbial analyses were carried out to study the microbiota of the intestine. Longitudinal 16S characterization was conducted on 4 paired samples from 60 mice, 240 samples in total. Each mouse was sampled prior to diet change, 1 week post diet change, 4 weeks post diet change and at termination, thus ensuring a thorough characterization of the faecal microbiota as a result of defensin treatment.

### Results

Weight change - hBD2. The standard high fat diet (HFD) fed groups had an equal food intake throughout the entire study period and had the same weight development with equal fat and lean mass the first 13 weeks, thus having the same starting point prior to the dietary intervention. The weight gain was significantly larger than in the low fat diet fed (LFD) group (*p<0.05 2-way ANOVA). After the dietary intervention the HFD groups continued to increase in weight, however the HFD plus hBD2 group tended to gain less weight the first 4 weeks post dietary intervention, although not significant (*p=0.07, 2-way ANOVA). From week 4 to the end of the study period the HFD plus hBD2 group gained similar weight as the standard HFD group (*p=0.82, 2-way ANOVA).

Weight change - HD5. All HFD fed groups had the same food intake during the study period and equal weight gain during the run-in period of 13 weeks. After dietary intervention the group fed HFD plus HD5 gained significantly less weight than the HFD control (*p<0.05, 2-way ANOVA). In addition, a tendency of decreasing fat percentage in the HFD plus HD5 group was observed, and a significantly lower fat percentage in the HFD plus HD5 was measured 4 weeks after dietary change in comparison to the HFD control (*p=0.009 2-way ANOVA).

### Microbiota.

Both defensins were shown to have a profound influence on the bacterial presence as well as bacterial absence (Figure 25). HD5 increased the abundance of Alloprevotella statistically significantly in the colon (p<0.02) (Figure 26) whereas hBD2 had no influence on Alloprevotella abundance. hBD2 dramatically and statistically significant increased the relative abundance of Bifidobacteriaceae both in the small intestine and in the colon (p<0.0001 and p<0.04 respectively; Figure 27). There was a trend towards HD5 increasing the abundance of Bifidobacteriaceae in the small intestine (Figure 27). Microbiota seems to play an important role in GVHD especially following the dramatic bacterial decontamination carried out just after the stem cell transplantation. Experimental treatment with faecal transplants are currently ongoing in a number of centers around the world e.g. with Prof Holler in Regensburg, Germany to try to reinstall a normal microbiota after the bacterial decontamination. This example 8 demonstrates that defensins have a profound influence on the microbiota composition in terms of number of species present and overall number of bacteria and thus seem to be able to treat a dysbiotic condition and reinstall a normal microbiota, which is key to preserve e.g. the colonic T reg cell homeostasis.

**Example 9** (for illustration).

To determine and assess the efficacy of prophylactic treatment with intranasal (IN) versus oral defensins in a murine house dust mite driven model of allergic asthma.

### Materials and methods

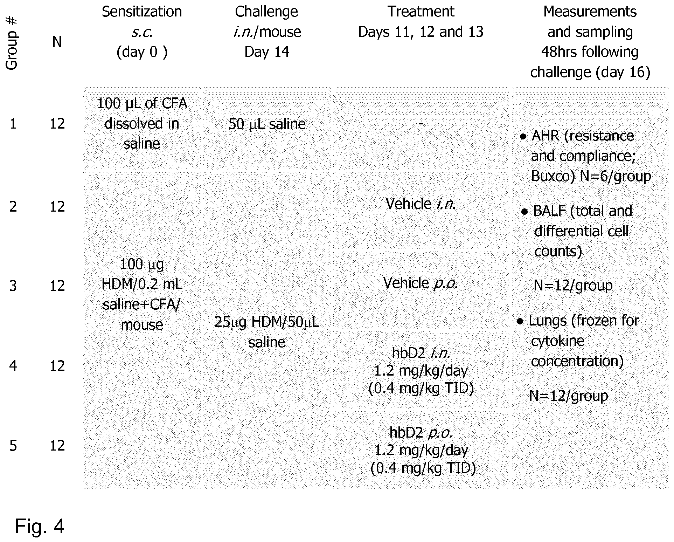

Treatment regime: Female 7-10 weeks old BALB/c mice were randomly allocated into 5 study groups one day prior to study start and subcutaneously (SC) sensitized to house dust mite (100 µg HDM in 200 µL saline plus Freund's complete adjuvant in 0.9% saline). The mice were treated with hBD2 orally and intranasally respectively at a dose of 1.2 mg/kg/day (0.4 mg/kg TID) starting on day 12 in the morning and continued TID at approximately 6 hours intervals. The last dose was administered on day 14 one hour prior to challenge. The total number of doses were 8 doses or a total of 2 mg/kg hBD2. Mice were then intranasally (IN) challenged with HDM on day 14 (HDM 25 µg in 50µL of saline) (Figure 4).

### Tests:

Airway inflammation: At 48 hours post challenge, bronchoalveolar lavage was performed washing the lungs with 3 volumes of cold PBS (0.4; 0.3 and 0.3 mL, total 1 mL). Total and differential leucocyte cell counts were determined on an automated haematological analyser Sysmex XT-2000iV.

Lung function: Starting 48 hours after HDM challenge, measurements of lung resistance and lung compliance were carried out after methacholine challenge (3.125 MCH1; 6.25 MCH2; 12.5 MCH3 and 25 mg/mL MCH4) by anaesthetized, cannulated mice using DSI's Buxco Finepoint RC system. Data are represented as airway resistance at 10 mg/kg methacholine and as dose responsive curves.

Lung sampling for cytokine analysis: After completion of every BAL, lungs were removed from the thorax, snap frozen in liquid nitrogen and stored frozen at -80 degrees Celcius until analysis of cytokine concentration of TNF-α, IL-4, IL-5, IL-6, IL-9, IL-13 and IL-33 in lung homogenate by ELISA.

### Results

An increase of lung resistance values and decrease of pulmonary compliance values in HDM-challenged vehicle treated animals in comparison to saline-challenged (non-asthmatic) mice was observed. An inflammatory response in both vehicle-treated groups of mice (oral and intranasal) was induced by a single HDM challenge 14 days post sensitization with HDM. It was characterized by a statistically significant increase in total cell, eosinophil, neutrophil, macrophage and lymphocyte counts in BALF (p<0.05) when compared to saline-challenged controls. Also, analysis of concentration of seven cytokines TNF-α, IL-4, IL-5, IL-6, IL-9, IL-13 and IL-33 in lung tissue homogenates revealed significantly higher levels in HDM-challenged animals compared to saline-challenged controls.

hBD2, both after oral and intranasal application TID, administered from day 12 to day 14 (a total of 2.0 mg/kg in 8 administrations), effectively inhibited the increase of airway resistance (Figure 28) and decrease of pulmonary compliance (Figure 29) as compared to HDM challenged vehicle treated animals. An effect on cellular influx in BALF was observed after oral application, that significantly inhibited neutrophil counts (Figure 30). A rebalance of the immune system with a complete normalization of cytokine concentrations in lung tissue homogenates was observed on TNF-α (Fig 31a), IL-4 (31b), L-5 (31c), IL-6 (31d), IL-9 (31e) and IL-13 (31f) cytokine levels following oral administration. There was a trend towards lowering of TNF-α, IL-4, IL-5, IL-6, IL-9 and IL-13 following intranasally administered hBD2, but this was not statistically significantly different from the control. All obtained results indicate clear preventive and anti-inflammatory effects of hBD2 in the house dust mite driven mouse model of allergic asthma.

The lung is the fourth organ involved in GVHD after the gut, the liver and the skin. This example demonstrates that defensins not only are able to preserve an intact immune system but crucially prevent a rise of a cytokine storm of all the key GVHD cytokines, TNF-α, IL-4, IL-5, IL-6, IL-9 and IL-13, that is a hallmark of acute GVHD. Inflammatory cytokine release or cytokine storm has been implicated as the primary mediator of the occurrence and severity of acute GVHD (Ball & Egeler, 2008).

**Example 10** (for illustration).

To determine and assess the efficacy of IN versus oral therapeutic intervention with defensins in a murine house dust mite model of allergic asthma.

### Materials and Methods

Treatment regime: Female 7-10 weeks old BALB/c mice were randomly allocated into 7 study groups one day prior to study start and subcutaneously (SC) sensitized to house dust mite (100 µg HDM in 200 µL saline plus Freund's complete adjuvant in 0.9% saline). Mice were then intranasally (IN) challenged with HDM on day 14 (HDM 25 µg in 50µL of saline). Dexamethasone was administered orally (1 mg/kg BID; 50µL phosphate buffered saline (PBS)) on day 14. hBD2 was administered IN or orally (1.7 mg/kg TID IN; 0.4 mg/kg TID IN; 0.4 mg/kg TID orally, 50 µL phosphate buffered saline) on day 14. The initial dose was administered 60 minutes prior to challenge, and the subsequent doses approximately 6 hours apart (Figure 5).

### Tests:

Airway inflammation: At 48 hours post challenge, bronchoalveolar lavage was performed washing the lungs with 3 volumes of cold PBS (0.4; 0.3 and 0.3 mL, total 1 mL). Total and differential leucocyte cell counts were determined on an automated haematological analyser Sysmex XT-2000iV.

Lung function: Starting 48 hours after HDM challenge, measurements of lung resistance and lung compliance were carried out after methacholine challenge (3.125 MCH1; 6.25 MCH2; 12.5 MCH3 and 25 mg/mL MCH4) by anaesthetized, cannulated mice using DSI's Buxco Finepoint RC system. Data are represented as airway resistance at 10 mg/kg methacholine and as dose responsive curves.

Lung sampling for cytokine analysis: After completion of every BAL, lungs were removed from the thorax, snap frozen in liquid nitrogen and stored frozen at -80 degrees Celcius until analysis of cytokine concentration of IL-1β, TNF-α, IL-6, IL-10 and IFNγ by ELISA.

### Results

An increase of lung resistance values and decrease of pulmonary compliance values in HDM-challenged vehicle treated animals in comparison to saline-challenged (non-asthmatic) mice was observed. An inflammatory response in both vehicle-treated groups of mice (oral and intranasal) was induced by a single HDM challenge 14 days post sensitization with HDM and adjuvant. It was characterized by a statistically significant increase in total cell, eosinophil, neutrophil, macrophage and lymphocyte counts in BALF (p<0.05) when compared to saline-challenged controls. Also, analysis of concentration of five cytokines IL-1β, TNF-α, IL-6, IL-10 and IFN-γ in lung tissue homogenates revealed significantly higher levels in HDM-challenged animals compared to saline-challenged controls.

Dexamethasone treatment significantly inhibited total cell and eosinophil counts but not neutrophil, macrophage and lymphocyte counts in BALF. In accordance with the cellular data, dexamethasone did not influence levels of IL-1β, TNF-α, IL-6, IL-10 and IFN-γ in lung tissue homogenates as compared to HDM/vehicle control. However, it influenced AHR measurements related to eosinophil counts. Obtained results indicate that this model is steroid resistant to a certain degree.

hBD2, both after oral and intranasal application TID, on day 14, effectively inhibited increase of airway resistance (Figure 32a and 32b) and decrease of pulmonary compliance (Figure 33a and 33b) as compared to HDM challenged vehicle treated animals. A more prominent effect was observed on some measured parameters after intranasal application, such as cellular influx in BALF, where both IN doses (0.4 mg/kg/day TID and 1.7 mg/kg/day TID) significantly inhibited total, neutrophil and macrophage cell counts (Figure 34 a, b and c) and a trend towards lowering of eosinophils, while the steroid standard dexamethasone failed to inhibit them. Similar significant effects were observed on IFN-γ (Fig. 35), IL-4 (Fig 37), IL-5 (Fig. 38), IL-6 (Fig. 39), IL-9 (Fig 40), and IL-10 (Fig 41) cytokine levels in lung tissue homogenates with both dosing routes. Perorally administered hBD2 significantly reduced TNF-α (Figure 36), while the intranasally administered hBD2 differed from the control but not significantly. All obtained results indicate clear anti-inflammatory effects of hBD2 in the house dust mite/Freund's complete adjuvant driven mouse model of allergic asthma. The lung is the fourth organ involved in GVHD after the gut, the liver and the skin. This example 10 shows that defensins not only are able to rebalance the immune system but crucially to treat or prevent a cytokine storm of key GVHD cytokines IFN-γ, TNF-α and IL-6, that is a hallmark of acute GVHD. In fact inflammatory cytokine release or cytokine storm has been implicated as the primary mediator of the occurrence and severity of acute GVHD (Ball & Egeler, 2008).

**Example 11** (for illustration).

To determine and assess the efficacy of IN versus oral therapeutic intervention with administration of defensins in a murine house dust mite model of allergic asthma.

### Materials and Methods

Treatment regime: Female 7-10 weeks old BALB/c mice randomly allocated into 4 study groups one day prior to study start were subcutaneously (SC) sensitized to house dust mite (100 µg HDM in 200 µL saline plus Freund's complete adjuvant in 0.9% saline). Mice were intranasally (IN) challenged with HDM on day 14 (HDM 25 µg in 50µL of saline). hBD2 was administered IN or orally (0.4 mg/kg TID IN; 0.4 mg/kg TID orally, 50 µL phosphate buffered saline) on day 14. The initial dose was administered 60 minutes prior to challenge, and the subsequent doses approximately 6 hours apart.

### Tests:

Lung tissue sampling: Lungs were removed from the thorax, snap frozen in liquid nitrogen and stored frozen at -80 degrees Celsius until analysis of cytokine concentration of IL-4, IL-5, IL-8 (KC), IL-9 and IL-13 in lung homogenate by ELISA. Lungs were inflated *in situ* in 10% buffered formalin, removed from thorax, placed individually in 10% buffered formalin, paraffin embedded *in toto,* sectioned and H&E/PAS stained.

Blood sampling: All terminal blood samples were collected via jugular vein bleeds. Blood was sampled to Li-heparin tubes, put on ice and immediately centrifuged at 4°C. Plasma was separated and stored at -80⁰C until the potential SCFA analysis.

Lung tissue sampling: The lungs were exposed and excised by gently opening the thorax and by cutting down either side of the sternum and ribs and trimming back. Lungs from the first 6 animals per group were removed from thorax, snap frozen in liquid nitrogen and stored frozen at -80°C until analysis of cytokine concentration by ELISA.

Lungs from the other 8 animals per group were inflated *in situ* with 10% buffered formalin, removed from thorax, placed individually in 10% buffered formalin, paraffin embedded *in toto,* sectioned and H&E/PAS stained. The paraffin blocks were retained for the IHC analysis.

### Read-outs

- Histopathology (H&E; PAS) (N=8/group; total N=32)
- Cytokines in lung tissue homogenates (IL-4, IL-5, IL-8 (KC), IL-9 and IL-13) (N=6/group; total N=24)

### Histopathology

Cellular influx (mononuclears, eosinophils, neutrophils) was assessed semi-quantitatively on H&E stained slides separately for peri-bronchial/bronchiolar and perivascular space as follows:
0 absent
1 few scattered inflammatory cells
2 larger aggregates
3 marked accumulation of cells

Overall score for inflammation was calculated as the sum of all individual scores. Goblet cell metaplasia, separately at a level of large airways and distal airways, was assessed at PAS-stained slides as follows:
0 no mucus containing cells along basement membrane
1 few positive cells along basement membrane with less than 75% of the cytoplasm stained
2 few positive cells along basement membrane with more than 75% of the cytoplasm stained
3 numerous positive cells along basement membrane with less than 75% of the cytoplasm stained
4 numerous positive cells along basement membrane with more than 75% of the cytoplasm stained

### Statistical evaluation

Data was processed using MS Excel. Statistical analysis was performed using GraphPad Prism software (version 5.04). Differences between groups are considered statistically significant when p<0.05.

Statistical analysis of selected histological score-values data was performed using median and non-parametric Mann-Whitney test.

### Results

An inflammatory response in both vehicle-treated groups of mice (oral and intranasal) was induced by a single HDM challenge 14 days post sensitization with HDM and adjuvant. It was characterized by a statistically significant increase in concentration of five cytokines IL-4, IL-5, IL-8, IL-9 and IL-13 in lung tissue homogenates and by severe histological inflammatory changes of lung tissue in HDM-challenged animals compared to saline-challenged controls.

hBD2, both after oral and intranasal application TID, on day 14, effectively inhibited the increase in histological inflammation of lung tissue as compared to HDM challenged vehicle treated animals (Figure 43, 44, 45). Significant effects were observed on IL-4, IL-5, IL-9 and IL-13 cytokine levels in lung tissue homogenates following oral administration and on IL-9 and IL-13 following IN administration. All obtained results indicate clear anti-inflammatory effects of hBD2 in the house dust mite/Freund's complete adjuvant driven mouse model of allergic asthma.

The lung is the fourth organ involved in GVHD after the gut, the liver and the skin. This example shows that defensins not only are able to rebalance the immune system but crucially to treat or prevent a cytokine storm of key GVHD cytokines IL-4, IL-5, IL-8, IL-9 and IL-13, that is a hallmark of acute GVHD. In fact inflammatory cytokine release or cytokine storm has been implicated as the primary mediator of the occurrence and severity of acute GVHD (Ball & Egeler, 2008).

### Example 12.

### Sequences

| SEQ ID NO: | Name | Sequence |
|---|---|---|
| 1 | hBD1 | DHYNCVSSGGQCLYSACPIFTKIQGTCYRGKAKCCK |
| 2 | hBD2 | GIGDPVTCLKSGAICHPVFCPRRYKQIGTCGLPGTKCC |
| 3 | hBD3 | GIINTLQKYYCRVRGGRCAVLSCLPKEEQIGKCSTRGR |
| 4 | hBD4 | ELDRICGYGTARCRKKCRSQEYRIGRCPNTYACCLRK |
| 5 | HD5 | ATCYCRTGRCATRESLSGVCEISGRLYRLCCR |
| 6 | HD6 | AFTCHCRRSCYSTEYSYGTCTVMGINHRFCCL |
| 7 | truncated hBD2 | PVTCLKSGAICHPVFCPRRYKQIGTCGLPGTKCCKKP |

### Example 13.

To determine and assess the efficacy of prophylactic treatment with oral hBD-2 in a murine model of acute graft versus host disease following stem cell transplantation.

### Materials and methods

Treatment regime: 12 female BALB/c mice were irradiated with 4.5 Gy (2 x 498 sec) at intervals of at least 4 hours on day 0. Bone marrow was harvested: hind limbs harvested from 2 female WT C57BL/6 mice aseptically. Skeletal muscle was removed and epiphysis cut away. Lumen of bone flushed with PBS and cells pelleted and taken up. Harvest of T-cells: spleens from 2 female WT C57BL/6 mice were meshed through a 100 µM cell strainer into a PBS filled dish. Cells were taken up in PBS and transferred into a 50 mL falcon tube and spun down. Cells were taken up in 1 mL PBS and 20 µL of CD4 microbead + 20 µL CD8 microbeads per spleen added for incubation for 20 min at 4 degrees C. Cells were washed with PBS and applied to MACS separation in order to positively select CD4+ and CD8+ cells. Positively selected T-cells were counted and taken up. Transplantation: directly after the second irradiation, all WT BALB/c recipient mice were injected with 5 x 1.000.000 BM cells (50 µL) i.v. into the retroorbital venous plexus under isoflurane anesthesia. 15 BALB/c mice were treated with 1.2 mg of hBD2/kg BW/day in 100 µL PBS per day vial oral gavage from day 0-10.

15 BALB/c mice received vehicle 100 µL PBS per day via oral gavage from day 0-10.

### Tests:

The mice were weighed daily for the initial 7 days and survival was monitored for 100 days.

### Results

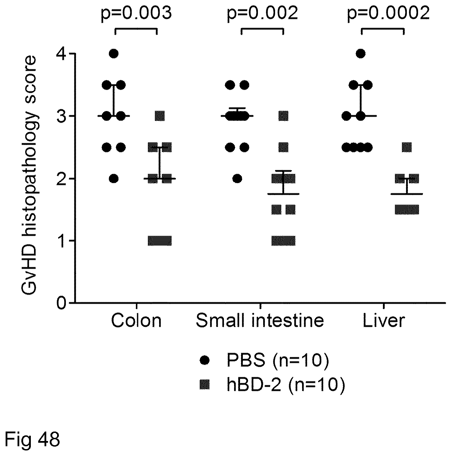

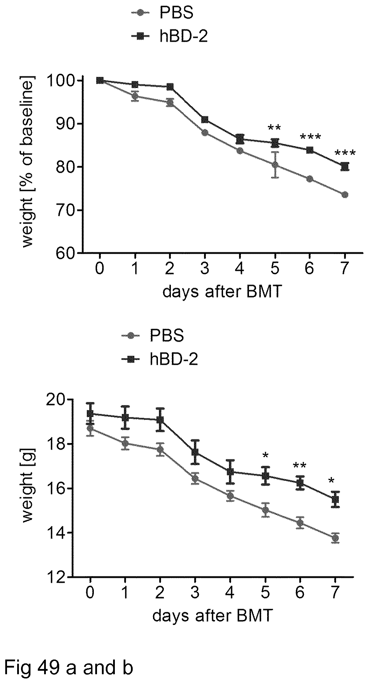

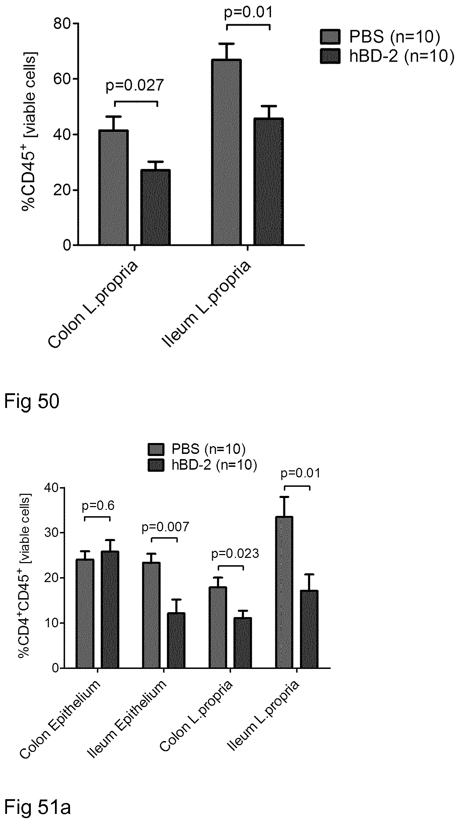

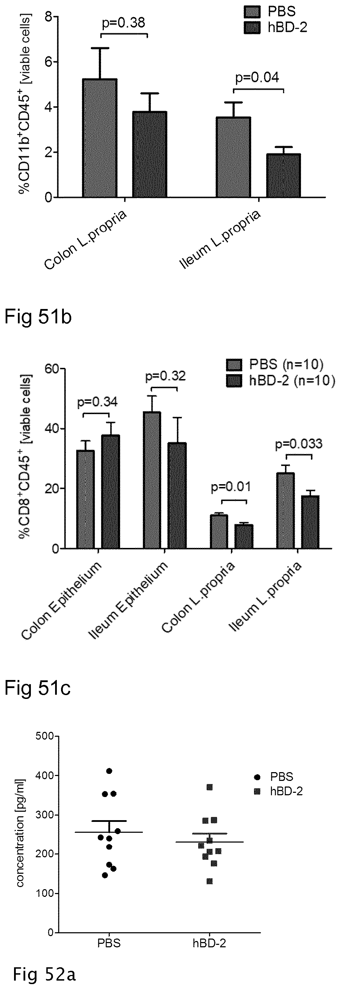

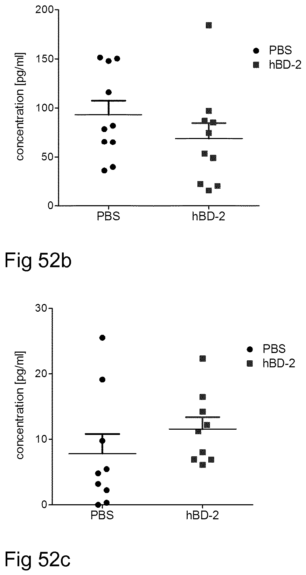

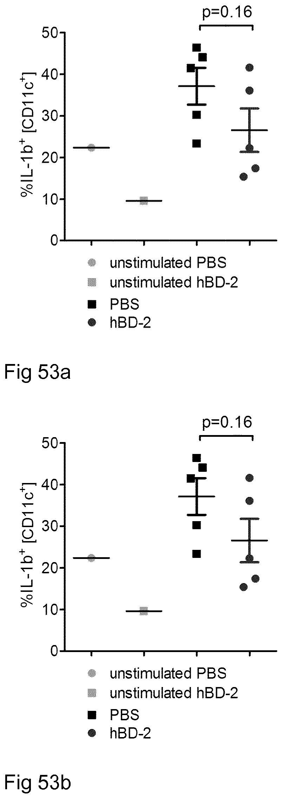

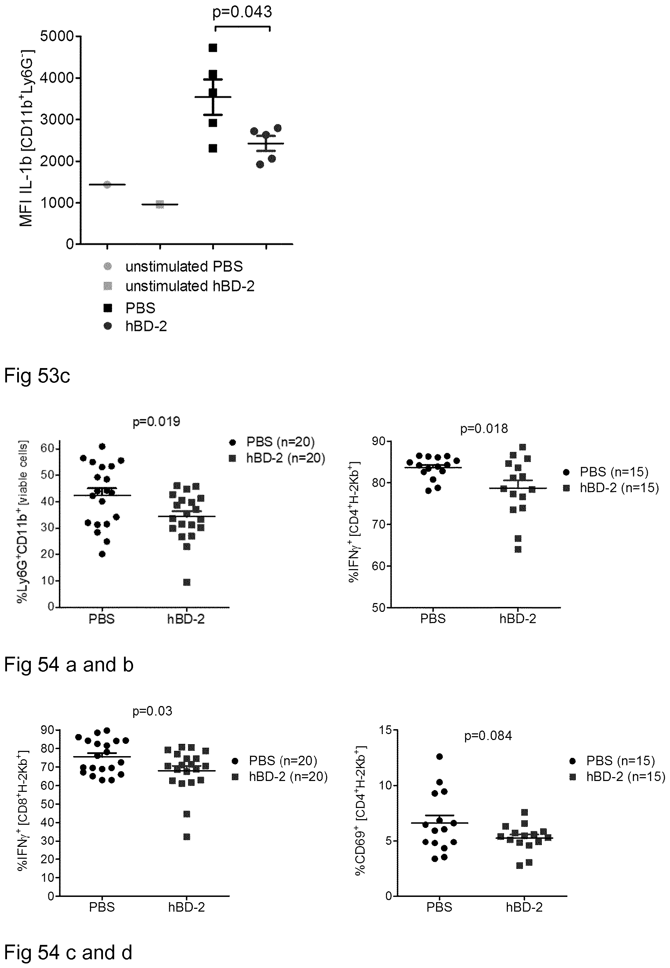

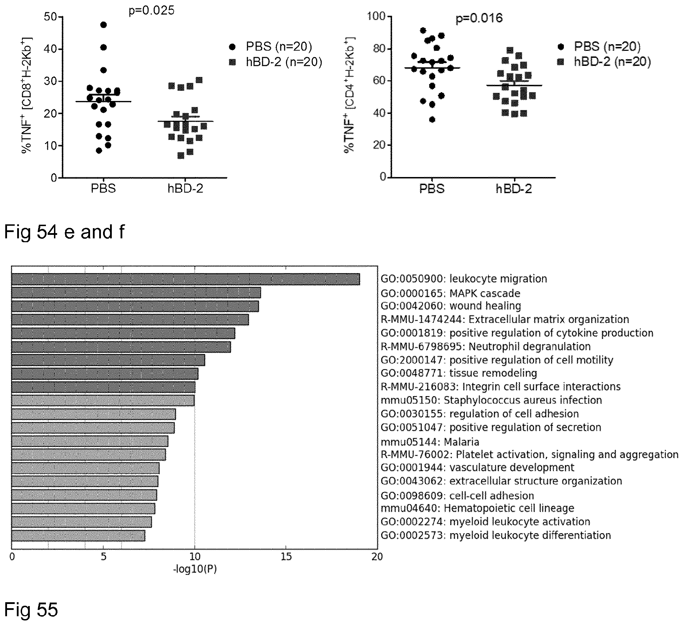

All 15 mice in the vehicle group had died by day 35 whereas only 4 or less than 30% of the hBD2 treated mice had died by day 35 and 8 mice were still alive by day 100 p<0.0001 (Figure 47). The histology score of the small intestine, colon and liver were all highly statistically lower for the hBD-2 treated group compared with PBS (Figure 48). The hBD2 treated mice lost statistically significantly less weight the first 7 days following bone marrow transplantation suggesting improved gut health and gut integrity (Figure 49). Treatment with hBD2 reduced the infiltration with CD45+ leucocytes (FACS analysis in Figure 50); intestinal T cell and myeloid cell infiltration (Figure 51 a-c) in lamina propria of the colon. Prophylactic treatment with hBD2 also showed reduction of TNF-α and IL-6 concentrations and increased concentration of IL-10 (Figure 52 a-c). The hBD-2 treatment additionally showed a reduction of IL-1β from myeloid cells (FACS analysis of the spleen in Figure 53 a-c). FACS analysis of the spleen also showed reduced number of neutrophils (Figure 54 a); reduced Th1 cytokine production of especially TNF-α and IFN-γ (Figure 54 b-f). Microarray analysis of the colon samples showed decreased inflammation, leucocyte and myeloid cell migration and tissue remodelling in the hBD-2 treated group versus PBS (Figure 55).

### Conclusion

This example 13 demonstrates that prophylactic treatment with hBD-2 for 10 days from the day of stem cell transplantation reduces mortality and increases long term survival dramatically.

### Example 14.

To determine and assess the efficacy of prophylactic treatment with oral hBD-2 versus cyclosporine in a murine model of acute graft versus host disease following stem cell transplantation.

### Materials and methods

Treatment regime: 20 female BALB/c mice were irradiated with 4.5 Gy (2 x 498 sec) at intervals of at least 4 hours on day 0. Bone marrow was harvested: hind limbs harvested from 2 female WT C57BL/6 mice aseptically. Skeletal muscle was removed and epiphysis cut away. Lumen of bone flushed with PBS and cells pelleted and taken up. Harvest of T-cells: spleens from 2 female WT C57BL/6 mice were meshed through a 100 µM cell strainer into a PBS filled dish. Cells were taken up in PBS and transferred into a 50 mL falcon tube and spun down. Cells were taken up in 1 mL PBS and 20 µL of CD4 microbead + 20 µL CD8 microbeads per spleen added for incubation for 20 min at 4 degrees C. Cells were washed with PBS and applied to MACS separation in order to positively select CD4+ and CD8+ cells. Positively selected T-cells were counted and taken up. Transplantation: directly after the second irradiation, all WT BALB/c recipient mice were injected with 5 x 1.000.000 BM cells (50 µL) i.v. into the retroorbital venous plexus under isoflurane anesthesia. 20 BALB/c mice were treated with 1.2 mg of hBD2/kg BW/day (n=7); 50 mg cyclosporine/kg BW on days 0, 3, 6 and 9 (n=7) or 100 µL PBS per day vial oral gavage (n=6) from day 0-10.

### Tests:

The mice were weighed at regular intervals during the study and survival was monitored for 90 days.

### Results

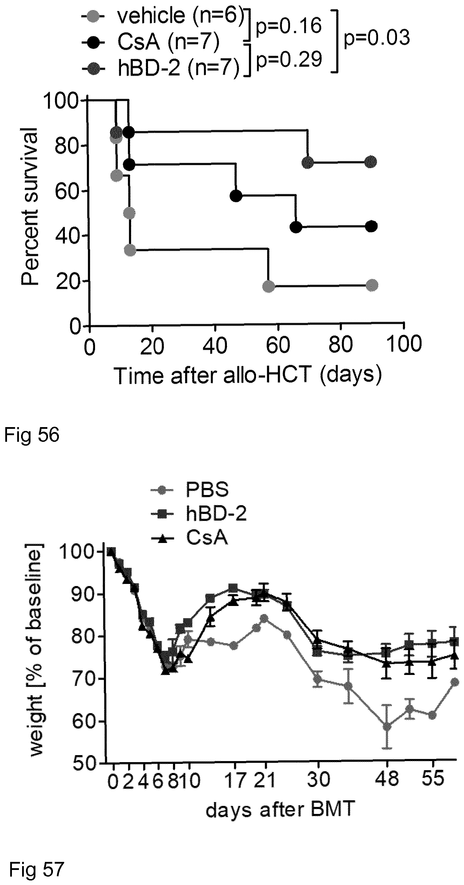

All but one of the PBS treated mice had died by day 90, whereas only 1 of the hBD-2 treated animals had died by day 90 (p = 0.03, Figure 56) and 3 animals of the cyclosporine treated animals had died (p=0.16, Figure 56). The hBD2 and cyclosporine treated mice lost statistically significantly less weight following bone marrow transplantation compared with the PBS treated mice suggesting improved gut health and gut integrity (Figure 57).

### Conclusion

This example 14 serves to demonstrate that prophylactic treatment with hBD-2 for 10 days from the day of stem cell transplantation reduces mortality at least on par with standard anti-GVHD treatment with cyclosporine.

### Example 15.

To determine and assess the efficacy of prophylactic treatment with oral HD5 in a murine model of acute graft versus host disease following stem cell transplantation.

### Materials and methods

Treatment regime: 22 female BALB/c mice were irradiated with 4.5 Gy (2 x 498 sec) at intervals of at least 4 hours on day 0. Bone marrow was harvested: hind limbs harvested from 2 female WT C57BL/6 mice aseptically. Skeletal muscle was removed and epiphysis cut away. Lumen of bone flushed with PBS and cells pelleted and taken up. Harvest of T-cells: spleens from 2 female WT C57BL/6 mice were meshed through a 100 µM cell strainer into a PBS filled dish. Cells were taken up in PBS and transferred into a 50 mL falcon tube and spun down. Cells were taken up in 1 mL PBS and 20 µL of CD4 microbead + 20 µL CD8 microbeads per spleen added for incubation for 20 min at 4 degrees C. Cells were washed with PBS and applied to MACS separation in order to positively select CD4+ and CD8+ cells. Positively selected T-cells were counted and taken up. Transplantation: directly after the second irradiation, all WT BALB/c recipient mice were injected with 5 x 1.000.000 BM cells (50 µL) i.v. into the retroorbital venous plexus under isoflurane anesthesia. 22 BALB/c mice were treated with 1.2 mg of HD5/kg BW/day; 1.2 mg of hBD-2/kg BW/day or 100 µL of PBS per day vial oral gavage from day 0-10.

### Tests:

Survival was monitored for 60 days.

### Results

Six out of eight of the PBS treated mice had died by day 60, whereas 2 out of 8 of the HD5 treated animals had died (p=0.06, Figure 58) and none of the hBD-2 treated animals had died by day 60 (p=0.008, Figure 58).

### Conclusion

This example 15 serves to demonstrate that prophylactic treatment for 10 days from the day of stem cell transplantation with either HD5 or hBD-2 reduces mortality dramatically.

**Example 16** (for illustration).

Anti microbial effect of orally administered defensins (HD5 and hBD-2).

### Method:

### Immunostaining of mucins and localization of bacteria by FISH

Mucus immunostaining was paired with fluorescent in situ hybridization (FISH), as previously described in order to analyze bacteria localization at the surface of the intestinal mucosa. Briefly, colonic tissues (proximal colon, 2nd cm from the cecum) without fecal material were placed in methanol-Carnoy's fixative solution (60% methanol, 30% chloroform, 10% glacial acetic acid) for a minimum of 3 hours at room temperature. Tissues were then washed in methanol 2 x 30 min, ethanol 2 x 15 min, ethanol/xylene (1:1), 15 min and xylene 2 x 15 min, followed by embedding in Paraffin with a vertical orientation. Five µm sections were performed and dewax by preheating at 60°C for 10 min, followed by xylene 60°C for 10 min, xylene for 10 min and 99.5% ethanol for 10 minutes. Hybridization step was performed at 50°C overnight with EUB338 probe (5'-GCTGCCTCCCGTAGGAGT-3', with a 5' labeling using Alexa 647) diluted to a final concentration of 10 µg/mL in hybridization buffer (20 mM Tris-HCI, pH 7.4, 0.9 M NaCl, 0.1% SDS, 20% formamide). After washing 10 min in wash buffer (20 mM Tris-HCI, pH 7.4, 0.9 M NaCl) and 3 x 10 min in PBS, PAP pen (Sigma-Aldrich) was used to mark around the section and block solution (5% fetal bovine serum in PBS) was added for 30 min at 4°C. Mucin- 2 primary antibody (rabbit H-300, Santa Cruz Biotechnology, Dallas, TX, USA) was diluted 1:1500 in block solution and apply overnight at 4°C. After washing 3 x 10 min in PBS, block solution containing anti-rabbit Alexa 488 secondary antibody diluted 1:1500, Phalloidin- Tetramethylrhodamine B isothiocyanate (Sigma-Aldrich) at 1µg/mL and Hoechst 33258 (Sigma- Aldrich) at 10µg/mL was applied to the section for 2h. After washing 3 x 10 min in PBS slides were mounted using Prolong anti-fade mounting media (Life Technologies, Carlsbad, CA, USA). Observations were performed with a Zeiss LSM 700 confocal microscope with software Zen 2011 version 7.1. This software was used to determine the distance between bacteria and epithelial cell monolayer, as well as the mucus thickness.

### Results:

A highly statistically significant difference in the lysis zone between the intestinal wall and the bacterial population was observed. The distance was small for mice fed both a low fat as well as a western diet, whereas HD5 and in particular hBD-2 had a profound effect on the distance (Figure 59).

### Conclusion

This experiment demonstrates that orally administered human HD5 and hBD-2 in a mouse exerts their microbiota modulating effect at the epithelial surface of the mouse as if they were produced by the epithelial cells of the mouse itself and not primarily in the lumen of the intestine as one would expect following oral administration.

### References

Ball, L.M. and Egeler, R.M. Acute GvHD: pathogenesis and classification. Bone Marrow Transplantation 2008; 41: S58-S64.
Donia, M.S. and Fischbach, M.A. 2015. Small molecules from the human microbiota. Science 349, 1254766
Dorrestein, P.C. et al., 2014. Finding the missing links among metabolites, microbes, and the host. Immunity 40: 824-832.
Ge et Ye. MPO is a potential biomarker of graft-versus-host-disease. Biomark Med 2013; 7: 391-393.
Henden and Hill. Cytokines in graft-versus-host-disease. J Immunol 2015; 194: 4604-4612.
Markey et al. The biology of graft-versus-host-disease: experimental systems instructing clinical practice. Blood 2014; 124: 354362.
Nalle and Turner. Intestinal barrier loss as a critical pathogenic link between inflammatory bowel disease and graft-versus-host-disease. Nature Mucosa Immunology 2015; 8: 720-730.
Salzman NH, Underwood MA and Bevins CL, 2007. Paneth cells, defensins, and the commensal microbiota: a hypothesis on intimate interplay at the intestinal mucosa.
Semin Immunol 19(2):70-83.
Schirmer, M. et al., 2016. Linking the human gut microbiome to inflammatory cytokine production capacity. Cell 167: 1125-1136
Teshima et al. Acute graft-versus-host-disese: Novel biological insights. Biol Blood Marrow Transplant 2016; 22: 53-58.
Trompette, A. et al., 2013. Gut microbiota metabolism of dietary fiber influences allergic airway disease and hematopoiesis. Nature Medicine 20: 159-168.
Wehkamp J, et al., 2002. Innate immunity and colonic inflammation: enhanced expression of epithelial alpha-defensins. Dig Dis Sci. 47(6):1349-55.
WO 2010/007166
WO 92/06204
WO 95/17413
WO 95/22625
US 5,223,409

## Claims

1. A human β-defensin selected from hBD-2 (SEQ ID NO.: 2), truncated hBD-2 (SEQ ID NO.: 7) or a functionally equivalent modified defensin comprising 1-4 amino acid modifications compared to hBD-2 of SEQ ID NO.: 2 or truncated hBD-2 of SEQ ID NO.: 7; wherein said modification(s) is a conservative amino acid substitution(s) or insertion(s) that do not significantly affect the folding of the defensin; or a single deletion(s), for use in the prevention or treatment of acute graft-versus-host-disease (GVHD) in a patient that has received or is about to receive an allogeneic hematopoietic stem cell transplantation.

2. The human β-defensin for use according to claim 1, wherein the human β-defensin is selected from hBD-2 (SEQ ID NO: 2), truncated hBD-2 (SEQ ID NO: 7) or a functionally equivalent modified defensin thereof comprising 1 amino acid modification compared to SEQ ID NO.: 2 or SEQ ID NO.: 7.

3. The human β-defensin for use according to any one of the preceding claims, wherein said human β-defensin is hBD2 (SEQ ID NO: 2) or truncated hBD2 (SEQ ID NO: 7).

4. The human β-defensin for use according to any one of preceding claims, wherein said defensin is comprised in a composition, wherein said composition comprises more than one defensin, such as two defensins, such as three defensins, such as four defensins, such as five defensins.

5. The human β-defensin for use according to claim 4, wherein said two defensins are hBD2 and HD5.

6. The human β-defensin for use according to any of the preceding claims, wherein administration of said human β-defensin to the patient is initiated on the day of allogenic hematopoietic stem cell transplantation, or is initiated when symptoms of GVHD appear after allogenic hematopoietic stem cell transplantation.

7. The human β-defensin for use according to any one of the preceding claims, wherein administration of human β-defensin is continued until the patient is free of symptoms caused by the allogenic hematopoietic stem cell transplantation, such as for at least one week, for example at least two weeks, such as at least 3 weeks, for example at least 4 weeks, such as at least 2 months, for example at least 3 months, at least 4 months, at least 6 months.

8. The human β-defensin for use according to any one of the preceding claims, wherein said human β-defensin further comprises at least one additional moiety selected from a group consisting of a cell penetrating peptide (CPP), an Albumin Binding Moiety (ABM), a detectable moiety (Z), and a half-life extending peptide.

9. The human β-defensin for use according to claim 8, wherein the at least one additional moiety is selected from the group consisting of a molecule capable of binding to a neonatal Fc receptor (FcRn), transferrin, albumin (HSA), XTEN or PEG, a homo-amino acid polymer (HAP), a proline-alanine-serine polymer (PAS), or an elastin-like peptide (ELP), hyaluronic acid, a negatively charged sialylated peptide such as the carboxy-terminal peptide (CTP) of chorionic gonadotropin (CG) β-chain, human IgG, and CH₃(CH₂)ₙCO- wherein n is 8 to 22.

10. The human β-defensin for use according to any one of the preceding claims, wherein the human β-defensin is administered in combination with prebiotics, probiotics, tryptophane, short chain fatty acids, glucocorticoids, cyclosporine, anti TNF-α, integrins, antibiotics, immunosuppressants, faecal transplantation, irradiation or a combination of these.

11. The human β-defensin for use according to any one of the preceding claims, wherein the daily dosage is between 0.1 and 10 mg defensin/kg, such as between 0.5 and 5 mg defensin/kg, such as between 1 and 2 mg defensin/kg, such as 1.2 mg defensin/kg per day.

12. The human β-defensin for use according to any of the preceding claims, wherein said human β-defensin is administered at least one time a day, such as at least twice a day, such as at least three times a day, such as at least four times a day, such as five times a day or continuously.

13. The human β-defensin for use according to any one of the preceding claims, wherein the administration is oral, buccal, sublingual, rectal, vaginal, intratracheal, intrapulmonary, intranasal, intracranial, subcutaneous, intravenous, dermal, or transdermal.

14. The human β-defensin for use according to any one of the preceding claims, wherein the patient has one or more symptoms selected from: severe intestinal and liver inflammation (e.g. **characterized by** abdominal pain, nausea, vomiting and jaundice) and loss of mucosal defense, cytokine storm, weight loss, sepsis, skin rash, itching, and redness.

15. The human β-defensin for use according to any one of the preceding claims, wherein the treatment
a. improves the symptoms as defined in claim 14;
b. decreases mortality and increases long term survival;
c. rebalances the immune system and prevents or treats the cytokine storm through normalization of the tissue cytokine production;
d. treats gut permeability and sepsis through normalization of the mucosal defense or myeloperoxidase activity in the intestines;
e. treats respiratory complications, lung capacity, lung inflammation and sepsis;
f. reduces histological lung inflammation, peribronchial and perivascular inflammation as well as inflammatory cell migration into bronchoalveolar lavage fluid;
g. treats or prevents inflammation and fibrosis of the lungs and skin, such as bronchiolitis obliterans and scleroderma;
h. increases gene richness, the number of phylae, the bacterial presence, the bacterial abundance and/or the short chain fatty acid production, and/or butyrate production;
i. decreases the acetate production from gut or lung microbiota of said patient;
j. matures, maintains or stabilizes a normal microbiota in the gut or lung or skin of said patient;
k. increases the abundance of *Allobaculum, Alloprevotella, Akkermansia, Barnesiella, Bifidobacteriaceae, Faecalibacterium, Lachnospira, Rothia* and *Veillonella* in the gut or lung of said patient; and/or
l. increases food uptake and weight gain in said patient.

## Patentansprüche

1. Humanes β-Defensin, ausgewählt aus hBD-2 (SEQ-ID-NR.: 2), trunkiertem hBD-2 (SEQ-ID-NR.: 7) oder einem funktionell äquivalenten modifizierten Defensin, umfassend 1-4 Aminosäuremodifikationen im Vergleich zu hBD-2 von SEQ-ID-NR.: 2 oder trunkiertem hBD-2 von SEQ-ID-NR: 7; wobei die Modifikation(en) eine konservative Aminosäuresubstitution(en) oder -insertion(en) ist/sind, die die Faltung des Defensins nicht signifikant beeinflussen; oder eine einzelne Deletion(en), zur Verwendung bei der Prävention oder Behandlung einer akuten Graft-versus-Host-Erkrankung (GvHD) bei einem Patienten, der eine allogene hämatopoetische Stammzelltransplantation erhalten hat oder in Kürze erhalten wird.

2. Humanes β-Defensin zur Verwendung nach Anspruch 1, wobei das humane β-Defensin ausgewählt ist aus hBD-2 (SEQ-ID-NR.: 2), trunkiertem hBD-2 (SEQ-ID-NR.: 7) oder einem funktionell äquivalenten modifizierten Defensin davon, umfassend 1 Aminosäuremodifikation im Vergleich zu SEQ-ID-NR.: 2 oder SEQ-ID-NR.: 7.

3. Humanes β-Defensin zur Verwendung nach einem der vorhergehenden Ansprüche, wobei das humane β-Defensin hBD2 (SEQ-ID-NR.: 2) oder trunkiertes hBD2 (SEQ-ID-NR.: 7) ist.

4. Humanes β-Defensin zur Verwendung nach einem der vorhergehenden Ansprüche, wobei das Defensin in einer Zusammensetzung umfasst ist, wobei die Zusammensetzung mehr als ein Defensin umfasst, wie zwei Defensine, wie drei Defensine, wie vier Defensine, wie fünf Defensine.

5. Humanes β-Defensin zur Verwendung nach Anspruch 4, wobei die beiden Defensine hBD2 und HD5 sind.

6. Humanes β-Defensin zur Verwendung nach einem der vorhergehenden Ansprüche, wobei die Verabreichung des humanen β-Defensins an den Patienten am Tag der allogenen hämatopoetischen Stammzelltransplantation eingeleitet wird, oder eingeleitet wird, wenn Symptome von GvHD nach allogener hämatopoetischer Stammzelltransplantation auftreten.

7. Humanes β-Defensin zur Verwendung nach einem der vorhergehenden Ansprüche, wobei die Verabreichung von humanem β-Defensin fortgesetzt wird, bis der Patient frei von Symptomen ist, die durch die allogene hämatopoetische Stammzelltransplantation verursacht werden, wie für mindestens eine Woche, zum Beispiel mindestens zwei Wochen, wie mindestens 3 Wochen, zum Beispiel mindestens 4 Wochen, wie mindestens 2 Monate, zum Beispiel mindestens 3 Monate, mindestens 4 Monate, mindestens 6 Monate.

8. Humanes β-Defensin zur Verwendung nach einem der vorhergehenden Ansprüche, wobei das humane β-Defensin ferner mindestens einen zusätzlichen Teil umfasst, der aus einer Gruppe ausgewählt ist, die aus einem Zell-penetrierenden Peptid (CPP), einem Albumin-bindenden Teil (ABM), einem nachweisbaren Teil (Z) und einem Halbwertszeit verlängernden Peptid besteht.

9. Humanes β-Defensin zur Verwendung nach Anspruch 8, wobei der mindestens ein zusätzlicher Teil ausgewählt ist aus der Gruppe, bestehend aus einem Molekül, das in der Lage ist, an einen neonatalen Fc-Rezeptor (FcRn), Transferrin, Albumin (HSA), XTEN oder PEG, ein Homo-Aminosäure-Polymer (HAP), ein Prolin-Alanin-Serin-Polymer (PAS) oder ein Elastin-ähnliches Peptid (ELP), Hyaluronsäure, ein negativ geladenes sialyliertes Peptid wie dem Carboxy-terminalen Peptid (CTP) der Chorionadotropin(CG)-β-Kette, von humanem IgG und CH₃CH₂)ₙCO-, wobei n 8 bis 22 ist, zu binden.

10. Humanes β-Defensin zur Verwendung nach einem der vorhergehenden Ansprüche, wobei das humane β-Defensin in Kombination mit Präbiotika, Probiotika, Tryptophan, kurzkettigen Fettsäuren, Glucocorticoiden, Cyclosporin, anti-TNF-alpha, Integrinen, Antibiotika, Immunsuppressiva, Fäkaltransplantation, Bestrahlung oder einer Kombination davon verabreicht wird.

11. Humanes β-Defensin zur Verwendung nach einem der vorhergehenden Ansprüche, wobei die Tagesdosis zwischen 0,1 und 10 mg Defensin/kg, wie zwischen 0,5 und 5 mg Defensin/kg, wie zwischen 1 und 2 mg Defensin/kg, wie 1,2 mg Defensin/kg pro Tag, liegt.

12. Humanes β-Defensin zur Verwendung nach einem der vorhergehenden Ansprüche, wobei das humane β-Defensin mindestens einmal pro Tag, wie mindestens zweimal pro Tag, wie mindestens dreimal pro Tag, wie mindestens viermal pro Tag, wie fünfmal pro Tag oder kontinuierlich, verabreicht wird.

13. Humanes β-Defensin zur Verwendung nach einem der vorhergehenden Ansprüche, wobei die Verabreichung oral, bukkal, sublingual, rektal, vaginal, intratracheal, intrapulmonal, intranasal, intrakranial, subkutan, intravenös, dermal oder transdermal ist.

14. Humanes β-Defensin zur Verwendung nach einem der vorhergehenden Ansprüche, wobei der Patient ein oder mehrere Symptome aufweist, ausgewählt aus: schwerer Darm- und Leberentzündung (z. B. **gekennzeichnet durch** Bauchschmerzen, Übelkeit, Erbrechen und Gelbsucht) und Verlust der Schleimhautabwehr, Zytokinsturm, Gewichtsverlust, Sepsis, Hautausschlag, Juckreiz und Rötung.

15. Humanes β-Defensin zur Verwendung nach einem der vorhergehenden Ansprüche, wobei die Behandlung
a. die Symptome, wie in Anspruch 14 definiert, verbessert;
b. die Mortalität senkt und das Langzeitüberleben erhöht;
c. das Immunsystem wieder ins Gleichgewicht bringt und den Zytokinsturm durch Normalisierung der Gewebezytokinproduktion verhindert oder behandelt;
d. die Darmpermeabilität und Sepsis durch Normalisierung der Schleimhautabwehr oder Myeloperoxidase-Aktivität im Darm behandelt;
e. Atemwegskomplikationen, Lungenkapazität, Lungenentzündung und Sepsis behandelt;
f. histologische Lungenentzündung, peribronchiale und perivaskuläre Entzündung sowie entzündliche Zellmigration in bronchoalveoläre Lavageflüssigkeit verringert;
g. Entzündungen und Fibrosen der Lunge und Haut, wie Bronchiolitis obliterans und Sklerodermie, behandelt oder verhindert;
h. den Genreichtum, die Anzahl der Phyla, die Bakterienpräsenz, die Abundanz von Bakterien und/oder die kurzkettige Fettsäureproduktion und/oder die Butyratproduktion erhöht;
i. die Azetatproduktion aus Darm- oder Lungenmikrobiom des Patienten verringert;
j. ein normales Mikrobiom im Darm oder in der Lunge oder Haut des Patienten reifen lässt, aufrechterhält oder stabilisiert;
k. die Abundanz von *Allobaculum, Alloprevotella, Akkermansia, Barnesiella, Bifidobacteriaceae, Faecalibacterium, Lachnospira, Rothia* und *Veillonella* im Darm oder in der Lunge des Patienten erhöht; und/oder
l. die Nahrungsaufnahme und Gewichtszunahme beim Patienten erhöht.

## Revendications

1. β-Défensine humaine choisie parmi la hBD-2 (SEQ ID NO. : 2), la hBD-2 tronquée (SEQ ID NO. : 7) ou une défensine modifiée fonctionnellement équivalente comprenant 1 à 4 modifications d'acide aminé par rapport à la hBD-2 de SEQ ID NO. : 2 ou la hBD-2 tronquée de SEQ ID NO. : 7 ; dans laquelle ladite ou lesdites modifications sont une ou plusieurs substitutions ou insertions d'acide aminé conservatrices qui n'affectent pas de manière significative le repliement de la défensine ; ou une ou plusieurs délétions uniques, pour une utilisation dans la prévention ou le traitement de la maladie aiguë du greffon contre l'hôte (GVHD) chez un patient qui a reçu ou est sur le point de recevoir une greffe de cellules souches hématopoïétiques allogéniques.

2. β-Défensine humaine pour une utilisation selon la revendication 1, dans laquelle la β-défensine humaine est choisie parmi la hBD-2 (SEQ ID NO : 2), la hBD-2 tronquée (SEQ ID NO : 7) ou une défensine modifiée fonctionnellement équivalente de celle-ci comprenant 1 modification d'acide aminé par rapport à SEQ ID NO : 2 ou SEQ ID NO : 7.

3. β-Défensine humaine pour une utilisation selon l'une quelconque des revendications précédentes, dans laquelle ladite β-défensine humaine est la hBD2 (SEQ ID NO : 2) ou la hBD2 tronquée (SEQ ID NO : 7).

4. β-Défensine humaine pour une utilisation selon l'une quelconque des revendications précédentes, dans laquelle ladite défensine est comprise dans une composition, dans laquelle ladite composition comprend plus d'une défensine, par exemple deux défensines, par exemple trois défensines, par exemple quatre défensines, par exemple cinq défensines.

5. β-Défensine humaine pour une utilisation selon la revendication 4, dans laquelle lesdites deux défensines sont la hBD2 et la HD5.

6. β-Défensine humaine pour une utilisation selon l'une quelconque des revendications précédentes, dans laquelle l'administration de ladite β-défensine humaine au patient est initiée le jour de la transplantation de cellules souches hématopoïétiques allogéniques, ou est initiée lorsque des symptômes de GVHD apparaissent après une transplantation de cellules souches hématopoïétiques allogéniques.

7. β-Défensine humaine pour une utilisation selon l'une quelconque des revendications précédentes, dans laquelle l'administration de β-défensine humaine est poursuivie jusqu'à ce que le patient soit exempt de symptômes causés par la transplantation de cellules souches hématopoïétiques allogéniques, par exemple pendant au moins une semaine, par exemple au moins deux semaines, par exemple au moins 3 semaines, par exemple au moins 4 semaines, par exemple au moins 2 mois, par exemple au moins 3 mois, au moins 4 mois, au moins 6 mois.

8. β-Défensine humaine pour une utilisation selon l'une quelconque des revendications précédentes, dans laquelle ladite β-défensine humaine comprend également au moins un groupement supplémentaire choisi dans un groupe constitué d'un peptide pénétrant dans les cellules (CPP), d'un groupement de liaison à l'albumine (ABM), d'un groupement détectable (Z) et d'un peptide prolongeant la demi-vie.

9. β-Défensine humaine pour une utilisation selon la revendication 8, dans laquelle l'au moins un groupement supplémentaire est choisi dans le groupe constitué par une molécule capable de se lier à un récepteur Fc néonatal (FcRn), la transferrine, l'albumine (HSA), XTEN ou PEG, un polymère d'acide homo-aminé (HAP), un polymère de proline-alanine-sérine (PAS) ou un peptide de type élastine (ELP), l'acide hyaluronique, un peptide sialylé chargé négativement tel que le peptide carboxy-terminal (CTP) de la chaîne β de la gonadotrophine chorionique (CG), l'IgG humaine et CH₃(CH₂)ₙCO-dans lequel n est de 8 à 22.

10. β-Défensine humaine pour une utilisation selon l'une quelconque des revendications précédentes, dans laquelle la β-défensine humaine est administrée en combinaison avec des prébiotiques, des probiotiques, du tryptophane, des acides gras à chaîne courte, des glucocorticoïdes, de la cyclosporine, des anti-TNF-α, des intégrines, des antibiotiques, des immunosuppresseurs, une transplantation fécale, une irradiation ou une combinaison de ceux-ci.

11. β-Défensine humaine pour l'utilisation selon l'une quelconque des revendications précédentes, dans laquelle la posologie quotidienne est comprise entre 0,1 et 10 mg de défensine/kg, telle qu'entre 0,5 et 5 mg de défensine/kg, telle qu'entre 1 et 2 mg de défensine/kg, telle que 1,2 mg de défensine/kg par jour.

12. β-Défensine humaine pour l'utilisation selon l'une quelconque des revendications précédentes, dans laquelle ladite β-défensine humaine est administrée au moins une fois par jour, par exemple au moins deux fois par jour, par exemple au moins trois fois par jour, par exemple au moins quatre fois par jour, par exemple cinq fois par jour.

13. β-défensine humaine pour une utilisation selon l'une quelconque des revendications précédentes, dans laquelle l'administration est orale, buccale, sublinguale, rectale, vaginale, intratrachéale, intrapulmonaire, intranasale, intracrânienne, sous-cutanée, intraveineuse, cutanée ou transdermique.

14. β-défensine humaine pour une utilisation selon l'une quelconque des revendications précédentes, dans laquelle le patient présente un ou plusieurs symptômes choisis parmi : une inflammation intestinale et hépatique sévère (par exemple **caractérisée par** des douleurs abdominales, des nausées, des vomissements et une jaunisse) et une perte de défense muqueuse, une tempête de cytokines, une perte de poids, une septicémie, une éruption cutanée, des démangeaisons et des rougeurs.

15. β-Défensine humaine pour une utilisation selon l'une quelconque des revendications précédentes, dans laquelle le traitement
a. améliore les symptômes tels que définis dans la revendication 14 ;
b. diminue la mortalité et augmente la survie à long terme ;
c. rééquilibre le système immunitaire et prévient ou traite la tempête de cytokines en normalisant la production de cytokines tissulaires ;
d. traite la perméabilité intestinale et la septicémie par la normalisation de la défense muqueuse ou de l'activité de la myéloperoxydase dans les intestins ;
e. traite les complications respiratoires, la capacité pulmonaire, l'inflammation pulmonaire et la septicémie ;
f. réduit l'inflammation histologique pulmonaire, l'inflammation péribronchique et périvasculaire ainsi que la migration des cellules inflammatoires dans le liquide de lavage bronchoalvéolaire ;
g. traite ou prévient l'inflammation et la fibrose des poumons et de la peau, comme la bronchiolite oblitérante et la sclérodermie ;
h. augmente la richesse génétique, le nombre de phylums, la présence bactérienne, l'abondance bactérienne et/ou la production d'acides gras à chaîne courte et/ou la production de butyrate ;
i. diminue la production d'acétate à partir du microbiote intestinal ou pulmonaire dudit patient ;
j. fait mûrir, maintient ou stabilise un microbiote normal dans l'intestin, les poumons ou la peau dudit patient ;
k. augmente l'abondance *d'Allobaculum, d'Alloprevotella, d'Akkermansia,* de *Barnesiella,* de *Bifidobacteriaceae,* de *Faecalibacterium,* de *Lachnospira,* de *Rothia* et de *Veillonella* dans l'intestin ou les poumons dudit patient ; et/ou
l. augmente l'absorption alimentaire et la prise de poids chez ce patient.
